# EUROPEAN PATENT APPLICATION

(11) **EP 1 741 721 A1**
(43) Date of publication of application: **10.01.2007**
(21) Application number: 05106200.8
(22) Date of filing: 07.07.2005
(51) Int. Cl.: C07K 14/38, C12N 15/62, C12N 15/80, C12N 1/15

(54) **Method for production of a compound in a eukaryotic cell**

(71) Applicant: DSM IP Assets B.V., 6411 TE Heerlen (NL)
(72) Inventor: Sagt, Cornelis, Maria, Jacobus, 3534 WE Utrecht (NL); Haaft, Ten, Petrus, Johannes, Fredrik, 2622 LD Delft (NL)
(74) Representative: Pallard, Caroline Chantal Patricia

(57) **Abstract**

The present invention relates to an eukaryotic cell containing peroxisomes that are capable to fuse with a membrane-structure of the cell involved in the secretory pathway of the cell. In this way, the eukaryotic cell is able to release the peroxisomal content outside the cell. The invention also relates to a method for production of a polypeptide of interest in said eukaryotic cell wherein said polypeptide of interest is present in the peroxisome of the cell. Said polypeptide will accumulate in the peroxisome by a signal promoting peroxisome localisation. Preferred host cells are filamentous fungal cells.

SEQUENCE LISTING

<110>  DSM IP Assets B.V.

<120>  METHOD FOR PRODUCTION OF A COMPOUND IN AN EUKARYOTIC CELL

<130>  24249EP/P1/

<160>  34

<170>  PatentIn version 3.1

<210>  1
<211>  36
<212>  DNA
<213>  Primer

<400>  1
gtcctgttaa ttaaccacca tggcgaacac aacgtg                                36

<210>  2
<211>  37
<212>  DNA
<213>  Primer

<400>  2
acttcaggcg cgcctcaaga gacatcactc attacgg                               37

<210>  3
<211>  45
<212>  DNA
<213>  Primer

<400>  3
acttcaggcg cgccttacag cttcgaagag acatcactca ttacg                      45

<210>  4
<211>  1793
<212>  DNA
<213>  Synthetic construct

<400>  4
gtcctgttaa ttaaccacca tggcgaacac aacgtgggag catagagcca agagcaagca      60

agctcagacg gcagcagcga ttccaccaga atggacttta ccagccgata tacttctcga     120

ctcctcggcg aatgtcctcg atgtgcccag gacctgtggg ctcctgaccg aaagagaaat     180

ccatattacc gaagactatg atgccacagc attacttgaa aagttggcta ccggagagtt     240

cagctcactc gaggtcacga cagcattctg caagcgggct gcaattgcgc agcagttgac     300

ctgctgcttg acggaaatct tcttcgacaa agcactagcc agagcaaagc agctcgacga     360

aattctggct caaactgggg taacaaccgg gccactgcat ggcctgccca taagtatcaa 420

ggagtcgttc aatgtccctg gagttccgac tacactgggg ttcgtcggat ttctcgatcg 480

ggccccggcg tccatgagtt ccgccctggt tgagatcctg aacaactgtg gggctgttct 540

atatgtcaag accaatgtcc cccagacaat gatgactccg gactcccata acaatgtctt 600

cggtcgtgtg ctcaacccgc acggacggag cctgaccgct ggaggcagca gtggcggaga 660

gggagcattg gtggcgatga ggggctcagt cctcggcgtg gggacagata tcgccggctc 720

cattcgcatc cccgccctct gctgtggtgt ttttggtttc aaacccacgg cttgccgtat 780

cccatatgca ggtcaaacgt ctgccgggcg tccgggtatg acgggcatcc tgccgtccgc 840

cgggcccatg tgccattcta tccgcgatgc agaactcttt ctcaaagttg tcctcaactc 900

gaggcctgcc gatctggatg actatgcact cgacattcca tggtcccctg ctccgcaaaa 960

ggagaggctt actattgggc tcctgcccga ggacccgtcg ttcccgcttc atccaccgat 1020

gcgacgtacc ctgaacgccg cagtgaaggc gctgactaca gccggacacc gggtcatcga 1080

cctctcagga caggctccgt ccttctccaa tgcatgttct ctggcattgc gatatttcgg 1140

cctggacccg gatcgaactg cactgcgcca cataacgcag gcgggcgaac ctttcattcc 1200

ttcgctcaaa ttcacctatg atctcaatga gccgacacgt gagcccacct tgagagatct 1260

attcgaatcg aatgtcgccc gtagtcagct cgcaacccag gcacgaaagc tcttcgttga 1320

taatcagctt gatctgttcc tgggcgcggc ttatcaaagc acatcggttc cccatgatac 1380

ctacggtatt ccggtatata ccgtgctttg gaacctgatt aatgtgggta tcccacactg 1440

cctttcttgt ttcttttttc tttttgtttt cccccccttc tttctgctcc aattgcatgg 1500

ggtgtctgct aatgatgcac tagtatccgg cgtgcgttat cccatttggg caggccaaca 1560

aggtagagga cgcagcgtat aaccgcgacg tctcttatgt gccagactgt aagttgcgcc 1620

cctcaattgg tctcgctcag tctactaact actgtagatc ggccagaaga aattgaaggc 1680

gcaccgtgtc acatccatct gataggcaga ccgatggcgg acgaaaaatt ggttcaggat 1740

gcgaagacta tatgcaccgt aatgagtgat gtctcttgag gcgcgcctga agt 1793

<210> 5
<211> 1802
<212> DNA
<213> Synthetic construct

<400> 5
gtcctgttaa ttaaccacca tggcgaacac aacgtgggag catagagcca agagcaagca 60

agctcagacg gcagcagcga ttccaccaga atggacttta ccagccgata tacttctcga 120

ctcctcggcg aatgtcctcg atgtgcccag gacctgtggg ctcctgaccg aaagagaaat 180

ccatattacc gaagactatg atgccacagc attacttgaa aagttggcta ccggagagtt 240

cagctcactc gaggtcacga cagcattctg caagcgggct gcaattgcgc agcagttgac 300

ctgctgcttg acggaaatct tcttcgacaa agcactagcc agagcaaagc agctcgacga 360

aattctggct caaactgggg taacaaccgg gccactgcat ggcctgccca taagtatcaa 420

ggagtcgttc aatgtccctg gagttccgac tacactgggg ttcgtcggat ttctcgatcg 480

ggccccggcg tccatgagtt ccgccctggt tgagatcctg aacaactgtg gggctgttct 540

atatgtcaag accaatgtcc cccagacaat gatgactccg gactcccata acaatgtctt 600

cggtcgtgtg ctcaacccgc acggacggag cctgaccgct ggaggcagca gtggcggaga 660

gggagcattg gtggcgatga ggggctcagt cctcggcgtg gggacagata tcgccggctc 720

cattcgcatc cccgccctct gctgtggtgt ttttggtttc aaacccacgg cttgccgtat 780

cccatatgca ggtcaaacgt ctgccgggcg tccgggtatg acgggcatcc tgccgtccgc 840

cgggcccatg tgccattcta tccgcgatgc agaactcttt ctcaaagttg tcctcaactc 900

gaggcctgcc gatctggatg actatgcact cgacattcca tggtcccctg ctccgcaaaa 960

ggagaggctt actattgggc tcctgcccga ggacccgtcg ttcccgcttc atccaccgat 1020

gcgacgtacc ctgaacgccg cagtgaaggc gctgactaca gccggacacc gggtcatcga 1080

cctctcagga caggctccgt ccttctccaa tgcatgttct ctggcattgc gatatttcgg 1140

cctggacccg gatcgaactg cactgcgcca cataacgcag gcgggcgaac ctttcattcc 1200

ttcgctcaaa ttcacctatg atctcaatga gccgacacgt gagcccacct tgagagatct 1260

attcgaatcg aatgtcgccc gtagtcagct cgcaacccag gcacgaaagc tcttcgttga 1320

taatcagctt gatctgttcc tgggcgcggc ttatcaaagc acatcggttc cccatgatac 1380

ctacggtatt ccggtatata ccgtgctttg gaacctgatt aatgtgggta tcccacactg 1440

cctttcttgt tttctttttc tttttgtttc ccccccttc tttctgctcc aattgcatgg 1500

ggtgtctgct aatgatgcac tagtatccgg cgtgcgttat cccatttggg caggccaaca 1560

aggtagagga cgcagcgtat aaccgcgacg tctcttatgt gccagactgt aagttgcgcc 1620

cctcaattgg tctcgctcag tctactaact actgtagatc ggccagaaga aattgaaggc 1680

gcaccgtgtc acatccatct gataggcaga ccgatggcgg acgaaaaatt ggttcaggat 1740

gcgaagacta tatgcaccgt aatgagtgat gtctcttcga agctataagg cgcgcctgaa 1800

gt                                                                  1802

<210>  6
<211>  40
<212>  DNA
<213>  Primer

<400>  6
gtcctgttaa ttaaccacca tgcttccccc ctttgattac                          40

<210>  7
<211>  39
<212>  DNA
<213>  Primer

<400>  7
acttcaggcg cgccctagtc aagctcaatc aatgggtat                           39

<210>  8
<211>  49
<212>  DNA
<213>  Primer

<400>  8
acttcaggcg cgccttacag cttcgagtca agctcaatca atgggtatt                49

<210>  9
<211>  1987
<212>  DNA
<213>  Synthetic construct

<400>  9
gtcctgttaa ttaaccacca tgcttccccc ctttgattac tttacgtatc gtcgcatccg     60

gtaggttacc ccgtgtgcct tcacgcagca acttaagcag tcctacacag cccacatgca    120

acttagccga cggctcactg accattcaca gggacctcaa gaggagggaa cgggcggctc    180

gatttgcatc gctatctccc gattatcatg cgcccttctc ggctatcgac agggttatca    240

ttaacaagcc tatccaggac ttggtatatg aggtccagaa tgattccttg gcacctttag    300

atgtcctacg cacatacggc aaggttgcag tcaaggctca cgaaaagacc aattgcgtga    360

ctgagcttct attgcctgag gcagaatcat gggctcagtc cgaagtaaac ctaaaaggtc    420

ccctggcggg tgtgcctatc tctttgaaag actcggtgca agtcaaagga ttcgatatta    480

ctctggggta taccaagttc gcgtgtaaac cgtataagga ggatggtcca atggcaaagc    540

tgttgaagga tgctggtagg tgacctgtgc cgcgaaacat cacagaaagt atccctgcgc    600

taatgatgaa ctccatcagg tgcggtccca tatgcgaaaa cggcgctgcc cgtgacgctt 660

ctgtcgttcg aatcagcaaa cgctctttgg ggtcactgcc ttaacccaca tgtcccggaa 720

tactctcctg gcggctcgac gggtggtgaa ggtgctctgc tggctcttgg tggtcgcatc 780

ggtatcgggt cggatgtcgc tggctcggtt cgcgttcccg ctgcctggag cggcatctac 840

tccctccgct gtagtactgg ccgctggcct aaggtcggag tcaacaccag catggctggc 900

caggaaggtg ttgccagtgt cttcagtcct atggcccgta ctttgaacga tctcacctat 960

ttcaccaaag ctattgtcgg aatgaagcct tggaactacg accataccgt ccaccctatc 1020

tcctggagag aagatgagga aattgaagcc caaaacaaaa ggttgaggat cggcctcatg 1080

agcaacgatg gtaagttcca agagcgggga catcaaggac actacttggg cctttctgac 1140

cacttgcagg tgtcgttccc ccaacgccgg ccattgaacg tgctatttcc accacagtag 1200

ccgccctcac cgccgctggg catactgtat ctgagatcac accccccgct gcggctgaca 1260

cttttactgg tctctccctc gcctcgcagc tgctcaactc tgatgggtgc gtcacgttta 1320

actcgcatct gcacagcttt gagccatccg accctggtgc agatcagctg acgcggatat 1380

gcaatctgcc ccgtcctcta cgttatctct actacctttg ggttcggtac atccgacggg 1440

atgagaagtg ggcgacgctg atccgcgggt ttgcccccaa gtctgcggcg gagctttgga 1500

agctcactgc ccagcgggaa gctttccggg cgacctggca cagctggtgg gatgccgaga 1560

cgcagcagta cgatttcatc ctctgccccg tcaatgcgac gccggctctg ccccacaaag 1620

ccatgcacga tgcggtatcc tcatgcggat acacgttcct ctggaacctg ctggactaca 1680

cagccggtgt cgtgcctgtc tcgcacgtgg acgcgaagaa ggatgctctg tctggtccgt 1740

acaagaaggt gctgaaacag ctgggagcca gcaacgcggt ggcctacggt gcctggaagc 1800

actacgacgc ggcgaagatg gcgggattgc ctactgcagt gcaggtggtg ggacgcagat 1860

ggcaggaaga gaaggtgctt ggatacatgg cggcagttga ggaggccttg gagcagtatc 1920

aggacccggt aactggagaa ggggggaaat acccattgat tgagcttgac tagggcgcgc 1980

ctgaagt 1987

<210> 10
<211> 1996
<212> DNA
<213> Synthetic construct

<400> 10
gtcctgttaa ttaaccacca tgcttccccc ctttgattac tttacgtatc gtcgcatccg 60

gtaggttacc ccgtgtgcct tcacgcagca acttaagcag tcctacacag cccacatgca 120

acttagccga cggctcactg accattcaca gggacctcaa gaggagggaa cgggcggctc 180

gatttgcatc gctatctccc gattatcatg cgcccttctc ggctatcgac agggttatca 240

ttaacaagcc tatccaggac ttggtatatg aggtccagaa tgattccttg gcacctttag 300

atgtcctacg cacatacggc aaggttgcag tcaaggctca cgaaaagacc aattgcgtga 360

ctgagcttct attgcctgag gcagaatcat gggctcagtc cgaagtaaac ctaaaaggtc 420

ccctggcggg tgtgcctatc tctttgaaag actcggtgca agtcaaagga ttcgatatta 480

ctctggggta taccaagttc gcgtgtaaac cgtataagga ggatggtcca atggcaaagc 540

tgttgaagga tgctggtagg tgacctgtgc cgcgaaacat cacagaaagt atccctgcgc 600

taatgatgaa ctccatcagg tgcggtccca tatgcgaaaa cggcgctgcc cgtgacgctt 660

ctgtcgttcg aatcagcaaa cgctctttgg ggtcactgcc ttaacccaca tgtcccggaa 720

tactctcctg gcggctcgac gggtggtgaa ggtgctctgc tggctcttgg tggtcgcatc 780

ggtatcgggt cggatgtcgc tggctcggtt cgcgttcccg ctgcctggag cggcatctac 840

tccctccgct gtagtactgg ccgctggcct aaggtcggag tcaacaccag catggctggc 900

caggaaggtg ttgccagtgt cttcagtcct atggcccgta ctttgaacga tctcacctat 960

ttcaccaaag ctattgtcgg aatgaagcct tggaactacg accataccgt ccaccctatc 1020

tcctggagag aagatgagga aattgaagcc caaaacaaaa ggttgaggat cggcctcatg 1080

agcaacgatg gtaagttcca agagcgggga catcaaggac actacttggg cctttctgac 1140

cacttgcagg tgtcgttccc ccaacgccgg ccattgaacg tgctatttcc accacagtag 1200

ccgccctcac cgccgctggg catactgtat ctgagatcac accccccgct gcggctgaca 1260

cttttactgg tctctccctc gcctcgcagc tgctcaactc tgatgggtgc gtcacgttta 1320

actcgcatct gcacagcttt gagccatccg accctggtgc agatcagctg acgcggatat 1380

gcaatctgcc ccgtcctcta cgttatctct actacctttg ggttcggtac atccgacggg 1440

atgagaagtg ggcgacgctg atccgcgggt ttgcccccaa gtctgcggcg gagctttgga 1500

agctcactgc ccagcgggaa gctttccggg cgacctggca cagctggtgg gatgccgaga 1560

cgcagcagta cgatttcatc ctctgccccg tcaatgcgac gccggctctg ccccacaaag 1620

ccatgcacga tgcggtatcc tcatgcggat acacgttcct ctggaacctg ctggactaca 1680

cagccggtgt cgtgcctgtc tcgcacgtgg acgcgaagaa ggatgctctg tctggtccgt 1740

acaagaaggt gctgaaacag ctgggagcca gcaacgcggt ggcctacggt gcctggaagc 1800

actacgacgc ggcgaagatg gcgggattgc ctactgcagt gcaggtggtg ggacgcagat 1860

ggcaggaaga gaaggtgctt ggatacatgg cggcagttga ggaggccttg gagcagtatc 1920

aggacccggt aactggagaa ggggggaaat acccattgat tgagcttgac tcgaagctat 1980

aaggcgcgcc tgaagt 1996

<210> 11
<211> 628
<212> DNA
<213> Aspergillus niger

<400> 11
atgtccgagc agccctatga tccctacatc ccctctggcg ccaatggggc tggcgccggc 60

gccagcgctg cgcaaaatgg cgaccccagg acacgggaaa tcgacaaagt aagttgccga 120

aacgcctcgc ggtcaacttt tatcgttcca caaggatatg tcgcccaggt tgaataggat 180

gtgatggctt tccagttttc atttggactt ccatctgagt tcaactggac ttggaggcca 240

ccgggtcttt ttgaatctta ttaagcttgt gtctattatg gcatttgtcg caaagtattt 300

actaacgctg tgtttcttct ctgtctagaa aatccaagaa accgttgaca caatgcgctc 360

caacatcttt aaagtttcag aacgtggtga acgtctagat tccctccagg acaagacgga 420

caatttggca acatcagcgc agggattccg cagaggtgcc aaccgcgtga ggaagcaaat 480

gtggtggaag gatatgaaga tgcgcgtgtg cctaatcgtt tgtatcatta ttctgctcat 540

tgtgattatc gtccctgcag gtaagatcag cgctatcctc cagcactcca ccaagctgga 600

ctcggaaaaa catcccggaa tgcgctaa 628

<210> 12
<211> 408
<212> DNA
<213> Aspergillus niger

<400> 12
atgtccgagc agccctatga tccctacatc ccctctggcg ccaatggggc tggcgccggc 60

gccagcgctg cgcaaaatgg cgaccccagg acacgggaaa tcgacaaaaa aatccaagaa 120

accgttgaca caatgcgctc caacatcttt aaagtttcag aacgtggtga acgtctagat 180

tccctccagg acaagacgga caatttggca acatcagcgc agggattccg cagaggtgcc 240

aaccgcgtga ggaagcaaat gtggtggaag gatatgaaga tgcgcgtgtg cctaatcgtt 300

tgtatcatta ttctgctcat tgtgattatc gtccctgcag gtaagatcag cgctatcctc 360

cagcactcca ccaagctgga ctcggaaaaa catcccggaa tgcgctaa                408

<210>  13
<211>  135
<212>  PRT
<213>  Aspergillus niger

<400>  13

Met Ser Glu Gln Pro Tyr Asp Pro Tyr Ile Pro Ser Gly Ala Asn Gly
1               5                   10                  15

Ala Gly Ala Gly Ala Ser Ala Ala Gln Asn Gly Asp Pro Arg Thr Arg
            20                  25                  30

Glu Ile Asp Lys Lys Ile Gln Glu Thr Val Asp Thr Met Arg Ser Asn
        35                  40                  45

Ile Phe Lys Val Ser Glu Arg Gly Glu Arg Leu Asp Ser Leu Gln Asp
    50                  55                  60

Lys Thr Asp Asn Leu Ala Thr Ser Ala Gln Gly Phe Arg Arg Gly Ala
65                  70                  75                  80

Asn Arg Val Arg Lys Gln Met Trp Trp Lys Asp Met Lys Met Arg Val
                85                  90                  95

Cys Leu Ile Val Cys Ile Ile Ile Leu Leu Ile Val Ile Ile Val Pro
            100                 105                 110

Ala Gly Lys Ile Ser Ala Ile Leu Gln His Ser Thr Lys Leu Asp Ser
        115                 120                 125

Glu Lys His Pro Gly Met Arg
    130                 135

<210>  14
<211>  974
<212>  DNA
<213>  Aspergillus niger

<400>  14
atgtctgcca agttccagga tgaggccgtc acctcgatac gggaggacac aaaggaattg      60

gtgcacaagg ttggaaaccg gttgactggc gatggctatc tcgctgtagg ttttgcgcct     120

gtgtaatcac accccgaatg cgtgttttgc agtccactga ttgagacaat gcgcgtccgt     180

tatagctcta cctccgccaa ctgcagtcca accccctgcg cactaagatg ttgacctccg 240

gtgtcctgtc cagtctgcaa gaaatcctgg cctcgtggat cgcccatgat gtcagcaagc 300

acggtcacta cttcagcgcc cgcgtcccca aaatggccct ctacggaatg ttcatcagcg 360

ccccgctggg ccactttctc atcggaattc tgcagcgggt cttcgctggc cggactagca 420

tcaaggccaa gatcctgcaa attctcgcca gcaacttgtt ggtatgttcg atctgacact 480

ccccttctga cgtgcggctg gaatgctgac gcgacgcagg tctcccccat ccaaaacgcc 540

gtgtacctgt gctgcatggc cgttatcgcg ggcgcgcgca ccttccacca ggtccgcgct 600

accgtgcggg ccggtttcat gcccgtcatg aaggtcagct gggtcacctc gcccattgcg 660

ctggcctttg cccagaagtt cctccccgag cacacctggg tgcctttctt caacattgtc 720

gggttcgtca ttggaaccta cgtcaacacg cacaccaaga agaagcgtct tgaggctctc 780

cgcaaggtaa atcaactacg tgacgatccc gccgacgcga ccagtcgcta acctagcacg 840

acagcgctac gaccaacgcc gtggacccgg tagcgagtac gacaagggcg actaccggta 900

aacgatgtaa atatactgta cctagcttat gactaccgac tggttagtgt ggacttcccg 960

atatggtcgg ttag 974

<210> 15
<211> 675
<212> DNA
<213> Aspergillus niger

<400> 15
atgtctgcca agttccagga tgaggccgtc acctcgatac gggaggacac aaaggaattg 60

gtgcacaagg ttggaaaccg gttgactggc gatggctatc tcgctctcta cctccgccaa 120

ctgcagtcca accccctgcg cactaagatg ttgacctccg gtgtcctgtc cagtctgcaa 180

gaaatcctgg cctcgtggat cgcccatgat gtcagcaagc acggtcacta cttcagcgcc 240

cgcgtcccca aaatggccct ctacggaatg ttcatcagcg ccccgctggg ccactttctc 300

atcggaattc tgcagcgggt cttcgctggc cggactagca tcaaggccaa gatcctgcaa 360

attctcgcca gcaacttgtt ggtctccccc atccaaaacg ccgtgtacct gtgctgcatg 420

gccgttatcg cgggcgcgcg caccttccac caggtccgcg ctaccgtgcg ggccggtttc 480

atgcccgtca tgaaggtcag ctgggtcacc tcgcccattg cgctggcctt tgcccagaag 540

ttcctccccg agcacacctg ggtgcctttc ttcaacattg tcgggttcgt cattggaacc 600

tacgtcaaca cgcacaccaa gaagaagcgt cttgaggctc tccgcaagtg tggacttccc 660

gatatggtcg gttag                                                      675

<210>  16
<211>  224
<212>  PRT
<213>  Aspergillus niger

<400>  16

Met Ser Ala Lys Phe Gln Asp Glu Ala Val Thr Ser Ile Arg Glu Asp
1               5                   10                  15

Thr Lys Glu Leu Val His Lys Val Gly Asn Arg Leu Thr Gly Asp Gly
            20                  25                  30

Tyr Leu Ala Leu Tyr Leu Arg Gln Leu Gln Ser Asn Pro Leu Arg Thr
        35                  40                  45

Lys Met Leu Thr Ser Gly Val Leu Ser Ser Leu Gln Glu Ile Leu Ala
    50                  55                  60

Ser Trp Ile Ala His Asp Val Ser Lys His Gly His Tyr Phe Ser Ala
65                  70                  75                  80

Arg Val Pro Lys Met Ala Leu Tyr Gly Met Phe Ile Ser Ala Pro Leu
                85                  90                  95

Gly His Phe Leu Ile Gly Ile Leu Gln Arg Val Phe Ala Gly Arg Thr
            100                 105                 110

Ser Ile Lys Ala Lys Ile Leu Gln Ile Leu Ala Ser Asn Leu Leu Val
        115                 120                 125

Ser Pro Ile Gln Asn Ala Val Tyr Leu Cys Cys Met Ala Val Ile Ala
    130                 135                 140

Gly Ala Arg Thr Phe His Gln Val Arg Ala Thr Val Arg Ala Gly Phe
145                 150                 155                 160

Met Pro Val Met Lys Val Ser Trp Val Thr Ser Pro Ile Ala Leu Ala
                165                 170                 175

Phe Ala Gln Lys Phe Leu Pro Glu His Thr Trp Val Pro Phe Phe Asn
            180                 185                 190

Ile Val Gly Phe Val Ile Gly Thr Tyr Val Asn Thr His Thr Lys Lys
        195                 200                 205

Lys Arg Leu Glu Ala Leu Arg Lys Cys Gly Leu Pro Asp Met Val Gly
    210                 215                 220

<210>  17
<211>  39
<212>  DNA
<213>  Primer

<400>  17
tgcttaatta accaccatgt ccgagcagcc ctatgatcc                              39

<210>  18
<211>  42
<212>  DNA
<213>  Primer

<400>  18
cctcatcctg gaacttggca gagcgcatct tcatatcctt cc                          42

<210>  19
<211>  543
<212>  DNA
<213>  Synthetic construct

<400>  19
tgcttaatta accaccatgt ccgagcagcc ctatgatccc tacatcccct ctggcgccaa      60

tggggctggc gccggcgcca gcgctgcgca aaatggcgac cccaggacac gggaaatcga     120

caaagtaagt tgccgaaacg cctcgcggtc aacttttatc gttccacaag gatatgtcgc     180

ccaggttgaa taggatgtga tggctttcca gttttcattt ggacttccat ctgagttcaa     240

ctggacttgg aggccaccgg gtctttttga atcttattaa gcttgtgtct attatggcat     300

ttgtcgcaaa gtatttacta acgctgtgtt tcttctctgt ctagaaaatc caagaaaccg     360

ttgacacaat gcgctccaac atctttaaag tttcagaacg tggtgaacgt ctagattccc     420

tccaggacaa gacggacaat ttggcaacat cagcgcaggg attccgcaga ggtgccaacc     480

gcgtgaggaa gcaaatgtgg tggaaggata tgaagatgcg ctctgccaag ttccaggatg     540

agg                                                                   543

<210>  20
<211>  42

<212>  DNA
<213>  Primer

<400>  20
ggaaggatat gaagatgcgc tctgccaagt tccaggatga gg                          42


<210>  21
<211>  35
<212>  DNA
<213>  Primer

<400>  21
attggcgcgc cctaaccgac catatcggga agtcc                                  35


<210>  22
<211>  1002
<212>  DNA
<213>  Synthetic construct

<400>  22
ggaaggatat gaagatgcgc tctgccaagt tccaggatga ggccgtcacc tcgatacggg      60

aggacacaaa ggaattggtg cacaaggttg gaaaccggtt gactggcgat ggctatctcg     120

ctgtaggttt tgcgcctgtg taatcacacc ccgaatgcgt gttttgcagt ccactgattg     180

agacaatgcg cgtccgttat agctctacct ccgccaactg cagtccaacc ccctgcgcac     240

taagatgttg acctccggtg tcctgtccag tctgcaagaa atcctggcct cgtggatcgc     300

ccatgatgtc agcaagcacg gtcactactt cagcgcccgc gtccccaaaa tggccctcta     360

cggaatgttc atcagcgccc cgctgggcca ctttctcatc ggaattctgc agcgggtctt     420

cgctggccgg actagcatca aggccaagat cctgcaaatt ctcgccagca acttgttggt     480

atgttcgatc tgacactccc cttctgacgt gcggctggaa tgctgacgcg acgcaggtct     540

cccccatcca aaacgccgtg tacctgtgct gcatggccgt tatcgcgggc gcgcgcacct     600

tccaccaggt ccgcgctacc gtgcgggccg gtttcatgcc cgtcatgaag gtcagctggg     660

tcacctcgcc cattgcgctg gcctttgccc agaagttcct ccccgagcac acctgggtgc     720

ctttcttcaa cattgtcggg ttcgtcattg gaacctacgt caacacgcac accaagaaga     780

agcgtcttga ggctctccgc aaggtaaatc aactacgtga cgatcccgcc gacgcgacca     840

gtcgctaacc tagcacgaca gcgctacgac caacgccgtg gacccggtag cgagtacgac     900

aagggcgact accggtaaac gatgtaaata tactgtacct agcttatgac taccgactgg     960

ttagtgtgga cttcccgata tggtcggtta gggcgcgcca at                       1002

<210>  23
<211>  1503
<212>  DNA
<213>  Synthetic construct

<400>  23
tgcttaatta accaccatgt ccgagcagcc ctatgatccc tacatcccct ctggcgccaa      60

tggggctggc gccggcgcca gcgctgcgca aaatggcgac cccaggacac gggaaatcga     120

caaagtaagt tgccgaaacg cctcgcggtc aacttttatc gttccacaag gatatgtcgc     180

ccaggttgaa taggatgtga tggctttcca gttttcattt ggacttccat ctgagttcaa     240

ctggacttgg aggccaccgg gtctttttga atcttattaa gcttgtgtct attatggcat     300

ttgtcgcaaa gtatttacta acgctgtgtt tcttctctgt ctagaaaatc caagaaaccg     360

ttgacacaat gcgctccaac atctttaaag tttcagaacg tggtgaacgt ctagattccc     420

tccaggacaa gacggacaat ttggcaacat cagcgcaggg attccgcaga ggtgccaacc     480

gcgtgaggaa gcaaatgtgg tggaaggata tgaagatgcg ctctgccaag ttccaggatg     540

aggccgtcac ctcgatacgg gaggacacaa aggaattggt gcacaaggtt ggaaaccggt     600

tgactggcga tggctatctc gctgtaggtt ttgcgcctgt gtaatcacac cccgaatgcg     660

tgttttgcag tccactgatt gagacaatgc gcgtccgtta tagctctacc tccgccaact     720

gcagtccaac cccctgcgca ctaagatgtt gacctccggt gtcctgtcca gtctgcaaga     780

aatcctggcc tcgtggatcg cccatgatgt cagcaagcac ggtcactact tcagcgcccg     840

cgtccccaaa atggccctct acggaatgtt catcagcgcc ccgctgggcc actttctcat     900

cggaattctg cagcgggtct tcgctggccg gactagcatc aaggccaaga tcctgcaaat     960

tctcgccagc aacttgttgg tatgttcgat ctgacactcc ccttctgacg tgcggctgga    1020

atgctgacgc gacgcaggtc tcccccatcc aaaacgccgt gtacctgtgc tgcatggccg    1080

ttatcgcggg cgcgcgcacc ttccaccagg tccgcgctac cgtgcgggcc ggtttcatgc    1140

ccgtcatgaa ggtcagctgg gtcacctcgc ccattgcgct ggcctttgcc cagaagttcc    1200

tccccgagca cacctgggtg cctttcttca acattgtcgg gttcgtcatt ggaacctacg    1260

tcaacacgca caccaagaag aagcgtcttg aggctctccg caaggtaaat caactacgtg    1320

acgatcccgc cgacgcgacc agtcgctaac ctagcacgac agcgctacga ccaacgccgt    1380

ggacccggta gcgagtacga caagggcgac taccggtaaa cgatgtaaat atactgtacc    1440

tagcttatga ctaccgactg gttagtgtgg acttcccgat atggtcggtt agggcgcgcc    1500

aat                                                                  1503

<210>  24
<211>  318
<212>  PRT
<213>  Synthetic construct

<400>  24

Met Ser Glu Gln Pro Tyr Asp Pro Tyr Ile Pro Ser Gly Ala Asn Gly
1               5                   10                  15

Ala Gly Ala Gly Ala Ser Ala Ala Gln Asn Gly Asp Pro Arg Thr Arg
            20                  25                  30

Glu Ile Asp Lys Lys Ile Gln Glu Thr Val Asp Thr Met Arg Ser Asn
        35                  40                  45

Ile Phe Lys Val Ser Glu Arg Gly Glu Arg Leu Asp Ser Leu Gln Asp
    50                  55                  60

Lys Thr Asp Asn Leu Ala Thr Ser Ala Gln Gly Phe Arg Arg Gly Ala
65                  70                  75                  80

Asn Arg Val Arg Lys Gln Met Trp Trp Lys Asp Met Lys Met Arg Ser
                85                  90                  95

Ala Lys Phe Gln Asp Glu Ala Val Thr Ser Ile Arg Glu Asp Thr Lys
            100                 105                 110

Glu Leu Val His Lys Val Gly Asn Arg Leu Thr Gly Asp Gly Tyr Leu
        115                 120                 125

Ala Leu Tyr Leu Arg Gln Leu Gln Ser Asn Pro Leu Arg Thr Lys Met
    130                 135                 140

Leu Thr Ser Gly Val Leu Ser Ser Leu Gln Glu Ile Leu Ala Ser Trp
145                 150                 155                 160

Ile Ala His Asp Val Ser Lys His Gly His Tyr Phe Ser Ala Arg Val
                165                 170                 175

Pro Lys Met Ala Leu Tyr Gly Met Phe Ile Ser Ala Pro Leu Gly His
            180                 185                 190

Phe Leu Ile Gly Ile Leu Gln Arg Val Phe Ala Gly Arg Thr Ser Ile
        195                 200                 205

Lys Ala Lys Ile Leu Gln Ile Leu Ala Ser Asn Leu Leu Val Ser Pro
    210                 215                 220

Ile Gln Asn Ala Val Tyr Leu Cys Cys Met Ala Val Ile Ala Gly Ala
225                 230                 235                 240

Arg Thr Phe His Gln Val Arg Ala Thr Val Arg Ala Gly Phe Met Pro
                245                 250                 255

Val Met Lys Val Ser Trp Val Thr Ser Pro Ile Ala Leu Ala Phe Ala
            260                 265                 270

Gln Lys Phe Leu Pro Glu His Thr Trp Val Pro Phe Phe Asn Ile Val
        275                 280                 285

Gly Phe Val Ile Gly Thr Tyr Val Asn Thr His Thr Lys Lys Lys Arg
    290                 295                 300

Leu Glu Ala Leu Arg Lys Cys Gly Leu Pro Asp Met Val Gly
305                 310                 315

<210>  25
<211>  720
<212>  DNA
<213>  Synthetic construct

<400>  25
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtaa     720


<210>  26
<211>  239
<212>  PRT
<213>  Synthetic construct

<400>  26

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  27
<211>  34
<212>  DNA
<213>  Primer

<400>  27
gtcctgttaa ttaaccacca tggtgagcaa gggc                                34

<210>  28
<211>  40
<212>  DNA
<213>  Primer

<400>  28
ctcatcctgg aacttggcag acttgtacag ctcgtccatg                          40

<210>  29
<211>  758
<212>  DNA
<213>  Synthetic construct

<400>  29
   gtcctgttaa ttaaccacca tggtgagcaa gggcgaggag ctgttcaccg gggtggtgcc     60
   catcctggtc gagctggacg gcgacgtaaa cggccacaag ttcagcgtgt ccggcgaggg    120
   cgagggcgat gccacctacg gcaagctgac cctgaagttc atctgcacca ccggcaagct    180
   gcccgtgccc tggcccaccc tcgtgaccac cctgacctac ggcgtgcagt gcttcagccg    240
   ctaccccgac cacatgaagc agcacgactt cttcaagtcc gccatgcccg aaggctacgt    300
   ccaggagcgc accatcttct tcaaggacga cggcaactac aagacccgcg ccgaggtgaa    360
   gttcgagggc gacaccctgg tgaaccgcat cgagctgaag ggcatcgact tcaaggagga    420
   cggcaacatc ctggggcaca agctggagta caactacaac agccacaacg tctatatcat    480
   ggccgacaag cagaagaacg gcatcaaggt gaacttcaag atccgccaca acatcgagga    540
   cggcagcgtg cagctcgccg accactacca gcagaacacc cccatcggcg acggccccgt 600
   gctgctgccc gacaaccact acctgagcac ccagtccgcc ctgagcaaag accccaacga 660
   gaagcgcgat cacatggtcc tgctggagtt cgtgaccgcc gccgggatca ctctcggcat 720
   ggacgagctg tacaagtctg ccaagttcca ggatgagg 758

<210> 30
<211> 41
<212> DNA
<213> Primer

<400> 30
catggacgag ctgtacaagt ctgccaagtt ccaggatgag g 41

<210> 31
<211> 38
<212> DNA
<213> Primer

<400> 31
acttcaggcg cgccctaacc gaccatatcg ggaagtcc 38

<210> 32
<211> 1004
<212> DNA
<213> Synthetic construct

<400> 32
catggacgag ctgtacaagt ctgccaagtt ccaggatgag gccgtcacct cgatacggga 60

ggacacaaag gaattggtgc acaaggttgg aaaccggttg actggcgatg gctatctcgc 120

tgtaggtttt gcgcctgtgt aatcacaccc cgaatgcgtg ttttgcagtc cactgattga 180

gacaatgcgc gtccgttata gctctacctc cgccaactgc agtccaaccc cctgcgcact 240

aagatgttga cctccggtgt cctgtccagt ctgcaagaaa tcctggcctc gtggatcgcc 300

catgatgtca gcaagcacgg tcactacttc agcgcccgcg tccccaaaat ggccctctac 360

ggaatgttca tcagcgcccc gctgggccac tttctcatcg gaattctgca gcgggtcttc 420

gctggccgga ctagcatcaa ggccaagatc ctgcaaattc tcgccagcaa cttgttggta 480

tgttcgatct gacactcccc ttctgacgtg cggctggaat gctgacgcga cgcaggtctc 540

ccccatccaa aacgccgtgt acctgtgctg catggccgtt atcgcgggcg cgcgcacctt 600

ccaccaggtc cgcgctaccg tgcgggccgg tttcatgccc gtcatgaagg tcagctgggt 660

cacctcgccc attgcgctgg cctttgccca gaagttcctc cccgagcaca cctgggtgcc 720

tttcttcaac attgtcgggt tcgtcattgg aacctacgtc aacacgcaca ccaagaagaa 780

gcgtcttgag gctctccgca aggtaaatca actacgtgac gatcccgccg acgcgaccag 840

tcgctaacct agcacgacag cgctacgacc aacgccgtgg acccggtagc gagtacgaca 900

agggcgacta ccggtaaacg atgtaaatat actgtaccta gcttatgact accgactggt 960

tagtgtggac ttcccgatat ggtcggttag ggcgcgcctg aagt 1004

<210> 33
<211> 1721
<212> DNA
<213> Synthetic construct

<400> 33
gtcctgttaa ttaaccacca tggtgagcaa gggcgaggag ctgttcaccg gggtggtgcc 60

catcctggtc gagctggacg gcgacgtaaa cggccacaag ttcagcgtgt ccggcgaggg 120

cgagggcgat gccacctacg gcaagctgac cctgaagttc atctgcacca ccggcaagct 180

gcccgtgccc tggcccaccc tcgtgaccac cctgacctac ggcgtgcagt gcttcagccg 240

ctaccccgac cacatgaagc agcacgactt cttcaagtcc gccatgcccg aaggctacgt 300

ccaggagcgc accatcttct tcaaggacga cggcaactac aagacccgcg ccgaggtgaa 360

gttcgagggc gacaccctgg tgaaccgcat cgagctgaag ggcatcgact tcaaggagga 420

cggcaacatc ctggggcaca agctggagta caactacaac agccacaacg tctatatcat 480

ggccgacaag cagaagaacg gcatcaaggt gaacttcaag atccgccaca acatcgagga 540

cggcagcgtg cagctcgccg accactacca gcagaacacc cccatcggcg acggccccgt 600

gctgctgccc gacaaccact acctgagcac ccagtccgcc ctgagcaaag accccaacga 660

gaagcgcgat cacatggtcc tgctggagtt cgtgaccgcc gccgggatca ctctcggcat 720

ggacgagctg tacaagtctg ccaagttcca ggatgaggcc gtcacctcga tacgggagga 780

cacaaaggaa ttggtgcaca aggttggaaa ccggttgact ggcgatggct atctcgctgt 840

aggttttgcg cctgtgtaat cacaccccga atgcgtgttt tgcagtccac tgattgagac 900

aatgcgcgtc cgttatagct ctacctccgc caactgcagt ccaaccccct gcgcactaag 960

atgttgacct ccggtgtcct gtccagtctg caagaaatcc tggcctcgtg gatcgcccat 1020

gatgtcagca agcacggtca ctacttcagc gcccgcgtcc ccaaaatggc cctctacgga 1080

atgttcatca gcgccccgct gggccacttt ctcatcggaa ttctgcagcg ggtcttcgct 1140

ggccggacta gcatcaaggc caagatcctg caaattctcg ccagcaactt gttggtatgt 1200

tcgatctgac actccccttc tgacgtgcgg ctggaatgct gacgcgacgc aggtctcccc 1260

catccaaaac gccgtgtacc tgtgctgcat ggccgttatc gcgggcgcgc gcaccttcca 1320

ccaggtccgc gctaccgtgc gggccggttt catgcccgtc atgaaggtca gctgggtcac 1380

ctcgcccatt gcgctggcct ttgcccagaa gttcctcccc gagcacacct gggtgccttt 1440

cttcaacatt gtcgggttcg tcattggaac ctacgtcaac acgcacacca agaagaagcg 1500

tctcgaggct ctccgcaagg taaatcaact acgtgacgat cccgccgacg cgaccagtcg 1560

ctaacctagc acgacagcgc tacgaccaac gccgtggacc cggtagcgag tacgacaagg 1620

gcgactaccg gtaaacgatt taaatatact gtacctagct tatgactacc gactggttag 1680

tgtggacttc ccgatatggt cggttagggc gcgcctgaag t 1721

<210> 34
<211> 462
<212> PRT
<213> Synthetic construct

<400> 34

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Ala Lys Phe Gln Asp Glu Ala Val Thr Ser Ile Arg Glu Asp Thr Lys
                245                 250                 255

Glu Leu Val His Lys Val Gly Asn Arg Leu Thr Gly Asp Gly Tyr Leu
            260                 265                 270

Ala Leu Tyr Leu Arg Gln Leu Gln Ser Asn Pro Leu Arg Thr Lys Met
        275                 280                 285

Leu Thr Ser Gly Val Leu Ser Ser Leu Gln Glu Ile Leu Ala Ser Trp
    290                 295                 300

Ile Ala His Asp Val Ser Lys His Gly His Tyr Phe Ser Ala Arg Val
305                 310                 315                 320

Pro Lys Met Ala Leu Tyr Gly Met Phe Ile Ser Ala Pro Leu Gly His
                325                 330                 335

Phe Leu Ile Gly Ile Leu Gln Arg Val Phe Ala Gly Arg Thr Ser Ile
            340                 345                 350

Lys Ala Lys Ile Leu Gln Ile Leu Ala Ser Asn Leu Leu Val Ser Pro
        355                 360                 365

Ile Gln Asn Ala Val Tyr Leu Cys Cys Met Ala Val Ile Ala Gly Ala
    370                 375                 380

Arg Thr Phe His Gln Val Arg Ala Thr Val Arg Ala Gly Phe Met Pro
385                 390                 395                 400

Val Met Lys Val Ser Trp Val Thr Ser Pro Ile Ala Leu Ala Phe Ala
                405                 410                 415

Gln Lys Phe Leu Pro Glu His Thr Trp Val Pro Phe Phe Asn Ile Val
            420                 425                 430

Gly Phe Val Ile Gly Thr Tyr Val Asn Thr His Thr Lys Lys Lys Arg
        435                 440                 445

Leu Glu Ala Leu Arg Lys Cys Gly Leu Pro Asp Met Val Gly
    450                 455                 460

## Description

### Field of the invention

The present invention relates to recombinant-DNA-technology. Specifically, this invention relates to a method for production of a compound in a eukaryotic cell, wherein the compound is present in a peroxisome capable of fusion with a membrane structure of the cell involved in the secretory pathway of the cell, causing the compound to be released outside of the cell.

### Background of the invention

Secretion of proteins by eukaryotic cells into the culture medium involves transfer of the proteins through the various membrane-enclosed compartments constituting the secretory pathway. First, the proteins are translocated into the lumen of the endoplasmic reticulum (ER). From there on, the proteins are transported in membrane vesicles to the Golgi complex and from the Golgi complex to plasma membrane. The secretory pathway involves several steps in which vesicles containing the secreted proteins are pinched off from the donor membrane, targeted to and fused with the acceptor membrane. At each of these steps, the function of several proteins such as chaperones or folding enzymes is needed in order to perform adequate maturation of the proteins including glycosylation and disulphide bridge formation. The extracellular proteins mature in the oxidizing environment of the ER where they become core glycosylated and this glycosylation process is subsequently completed in the Golgi complex.

Several attempts have been made to increase protein secretion in eukaryotes. A common approach to increase secretion of heterologous proteins is to use signal sequences (see for example EP 0215 594). The conventional secretory pathway in eukaryotic cells, as outlined above, is by definition adapted to the maturation of extracellular proteins. The maturation of intracellular proteins is realized in the reducing environment of the cytoplasm having specific chaperones and folding proteins. Production of proteins, especially intracellular proteins, in an industrial setting is still a difficult task due to the low yield of proteins caused by the inefficiency of both the secretion and the down stream processing (Hopkins TR. Physical and chemical cell disruption for the recovery of intracellular proteins. Bioprocess Technol. 1991;12:57-83.)

Due to the growing industrial importance to produce proteins and to the poor efficiency of both secretion and down stream processing pathways, there is still a need to obtain improved process for production of proteins in eukaryotic cells. The present invention provides a novel method to produce proteins with high efficiency.

### Description of the Figures

Figure 1 depicts the expression vector used to express the acetamidase gene in *A. niger.*
Figure 2 depicts the expression vector used to express the acetamidase gene in *K. lactis.*
Figure 3 depicts the acetamidase activity measured in control *A. niger* cells transformed with the acetamidase gene or in A. *niger* cells transformed with the acetamidase gene fused to the SKL sequence.
Figure 4 depicts the acetamidase activity measured in control *K. lactis* cells transformed with the acetamidase gene or in *K. lactis* cells transformed with the acetamidase gene fused to the SKL sequence.
Figure 5 shows the peroxisomes of *A. niger* transformed with GFP-SKL.
Figure 6 shows the peroxisomes of *A. niger* transformed with GFP-SKL and cultivated with Na-oleate, a mediator of peroxisome proliferation.
Figure 7 shows a SDS-PAGE gel containing culture supernatants of several transformed *A niger* strains that demonstrates the release of GFP-SKL in the supernatant.
Figure 8 shows a Western blot containing culture supernatants of several transformed *A. niger* strains that demonstrates the release of GFP-SKL in the supernatant.
Figure 9 shows *A*. *niger* strains expressing GFP (panel C), GFP-SKL (panel B) or GFP/Pmp22 (panel A).

### Detailed description of the invention

In a first aspect, the invention relates to a eukaryotic cell containing peroxisomes that are capable to fuse with a membrane-structure of the cell involved in the secretory pathway of the cell. In this way, the present invention provides a novel capability of eukaryotic cells in that they are capable to release the content of a peroxisome outside of the cell. In this way, polypeptides that otherwise would not be secreted by a eukaryotic cell can now be advantageously released via the peroxisomal route.

According to the invention fusion of a peroxisome with a membrane-structure of the cell involved in the secretory pathway of the cell is equivalent with fusion of the peroxisomal membrane with a membrane-structure of the cell involved in the secretory pathway of the cell. Fusion means the merging of diverse elements to form a unified whole: one element is here the peroxisomal membrane and other elements are membranes from membrane-structures of the cell involved in the secretory pathway of the cell. For example, if fusion of peroxisome with the plasma membrane occurs, the peroxisomal content will be directly exported outside of the cell. In another example, if the fusion of the peroxisome with the Golgi complex and/or with the ER occurs, the peroxisomal content will be translocated into the Golgi complex and/or the ER, thereby being indirectly exported outside of the cell via the endogenous secretory pathway of the cell.

A peroxisome (also called microbody) is defined as a single membrane bound organelle involved in a variety of metabolic processes ubiquitously found in eukaryotic cells (Sakai et al. Yeast 14, 1175-1187; 1998). In eukaryotic cells, peroxisomes normally do not fuse with a membrane-structure of the cell involved in the secretory pathway of the cell but are maintained as single membrane bound organelles within the cytosol.

In the context of the invention, a membrane-structure of the cell involved in the secretory pathway of the cell may be any membrane structure involved in the secretion of a polypeptide by the cell. Throughout this invention, the phrase "membrane-structure of the cell involved in the secretory pathway of the cell" and the term "membrane-structure" are used synonymously. Preferably, the membrane-structure of the cell involved in the secretory pathway of the cell is selected from the group consisting of the plasma membrane, the Golgi complex and the Endoplasmic Reticulum (ER). The Golgi complex is defined as to include minimally three (cis-, medial- and trans-Golgi) distinct flattened membrane-bound compartments (cisternae), which associate with each other to form a stack (Pfeffer SR, Constructing a Golgi complex. J Cell Biol. 2001 Dec 10;155(6):873-5).

The process of membrane fusion is highly conserved in all eukaryotic species. According to the present state of the art, the central components driving membrane fusion are polypeptides called SNAREs (Soluble N-ethylmaleimide-sensitive factor Attachment protein Receptors). Complementary SNAREs are present on donor and acceptor membranes and are distinguished by a conserved sequence motif (SNARE motif). To mediate membrane fusion, four SNARE motifs bundle to form a parallel coiled-coil structure, referred to as SNAREpin. The SNAREpin is comprised of at least one membrane-anchored SNARE on the acceptor membrane-structure and at least one membrane anchored SNARE on the donor membrane-structure. Soluble or alternatively membrane bound SNAREs containing one or two SNARE motifs (e.g. Sec9) may complement SNAREpin formation. The arrangement of SNAREpin components in the donor : acceptor membrane structures may be 1 : 3, but can also be 2 : 2. (Burri L, Lithgow T. A complete set of SNAREs in yeast. Traffic. 2004 Jan;5(1):45-52).

According to the invention, the fusion of the peroxisome membrane with a membrane-structure of the cell involved in the secretory pathway of the cell can be effectuated in several ways, which can be used alone or in combination.

The present invention disclose that to obtain fusion of a peroxisome with the plasma membrane of a eukaryotic cell, a fusing polypeptide or a part thereof is exposed at the surface of the peroxisome. According to the invention, a fusing polypeptide is a polypeptide involved in the fusion of a donor membrane-structure with an acceptor membrane-structure and normally exposed at the surface of the donor membrane-structure. A donor membrane-structure is defined as a membrane-structure that is capable to generate vesicles that are able to fuse with an acceptor membrane-structure. Preferably, the donor membrane structure is selected from the group consisting of the Golgi complex and the ER.

Fusing polypeptides typically comprise a part exposed at the surface of the donor membrane-structure, e.g. the Golgi complex or the ER, and a transmembrane domain. In a preferred embodiment, that part of the fusing polypeptide normally exposed at the surface of the donor membrane-structure, i.e. the surface exposed domain, is used for exposure at the surface of the peroxisome.

In a preferred embodiment, the donor membrane structure is the Golgi complex, i.e. the fusing polypeptide is a polypeptide normally expressed at the surface of a vesicle of the Golgi complex and known to be involved in the fusion of the Golgi vesicle with the plasma membrane.

Preferred fusing polypeptides are polypeptides of the family of v-SNARE polypeptides or v-SNAREs (vesicle-SNAREs, Jahn et al, Annu Rev. Biochem.(1999) 863-911; Burri et al. *supra*)*.*

Several v-SNAREs expressed at the surface of a vesicle of the Golgi complex have already been described, e.g. Snc1 and Snc2 (Burri et al. *supra*). Examples of a v-SNARE expressed at the surface of the ER are Sec22 and Ykt6 (Burri et al. *supra*)*.* In a preferred embodiment of the invention, at least one of the v-SNAREs Snc1 or Snc2, or a homologue thereof, is used. An example of a Snc1/Snc2 homologue is the *Aspergillus niger* SncA polypeptide as provided by the present invention.

The present invention encompasses the use of homologues of any polypeptide (or its encoding gene) that is described in the present invention with reference to its origin from a specific reference species, usually yeast (*S. cerevisiae*). Thus, a homologue (or a homologous sequence) is defined as a polypeptide from another species than the reference species that exerts substantially the same function as the polypeptide of the reference species, although the homologue may have a different name than the name used in the reference species. Typically, such a homologue may have a degree of identity with the polypeptide of the reference species of at least 50%. Such a homologue preferably originates from the same eukaryotic species as the eukaryotic cell that is modified according to the present invention.

To obtain exposure of a fusing polypeptide, such as a v-SNARE, at the peroxisomal surface, the fusing polypeptide or part thereof is operatively associated with, preferably fused to, a peroxisomal membrane polypeptide or part thereof. In this way a chimeric polypeptide is obtained comprising a fusing polypeptide part or component and a peroxisomal membrane polypeptide part or component.

The peroxisomal membrane polypeptide or part thereof, as component of the chimeric polypeptide, should be capable to mediate targeting to and localisation of the entire chimeric polypeptide in the peroxisomal membrane, thereby functioning as a membrane anchor and exposing the fusing polypeptide part of the chimeric polypeptide on the surface of the peroxisome. Preferably, the peroxisomal membrane polypeptide is trimmed at the N-terminal part to result in exposure of the fusing polypeptide as close as possible to the peroxisomal membrane, without abrogating peroxisomal targeting of the chimeric fusing polypeptide.

Preferably, that part of the fusing polypeptide, such as a v-SNARE, is used as component of the chimeric polypeptide that comprises at least the domain of the fusing polypeptide normally exposed at the surface of the donor membrane-structure, such as a Golgi vesicle. The transmembrane domain(s) of the fusing polypeptide may be absent in the chimeric polypeptide, either partly or completely.

Any peroxisomal membrane polypeptide or a part thereof suitable to function as a peroxisomal membrane anchor is suitable to be used for operative associated with, preferably fusion to, at least the surface-exposed domain of the fusing polypeptide, as long as the result is a chimeric peptide comprising the fusing polypeptide part located at the cytosolic side (or surface) of the peroxisome (able to interact with the membrane-structure of the cell involved in the secretory pathway of the cell). Preferably, the fusing polypeptide part is located at the N-terminal side of the chimeric polypeptide.

An example of a preferred peroxisomal membrane polypeptide is Peroxisomal Membrane Polypeptide 22 (Pmp22) or a homologue thereof (Brosius U, Dehmel T, Gartner J. Two different targeting signals direct human peroxisomal membrane protein 22 to peroxisomes. J Biol Chem. 2002 Jan 4;277(1):774-84). An example of a Pmp22 homologue is the *Aspergillus niger* Pmp22 as provided by the present invention. According to a preferred embodiment, the part of the Pmp22 polypeptide that is used as component of the chimeric polypeptide comprises at least one peroxisomal transmembrane domain of Pmp22, more preferably all peroxisomal transmembrane domains of Pmp22.

Preferably, Pmp22 is trimmed at the N-terminal part as described above. Said trimming comprises a deletion of at least the first methionine. The trimming on the N-terminal part may further comprise the deletion of the N-terminal 1 to 50, e.g. 48, amino acids, preferably of the N-terminal 1 to 35, e.g. 33, amino acids, more preferably of the N-terminal 1 to 20, e.g. 18, amino acids.

According to a preferred embodiment, the domain of a v-SNARE (e.g. Snc1, Snc2 or SncA) normally exposed at the surface of the donor membrane-structure is operatively associated with (fused to) the peroxisomal membrane polypeptide Pmp22, to decorate peroxisomes with the corresponding part of the v-SNARE, e.g. Snc1, Snc2 or SncA. More preferably, the surface exposed domain of SncA is used. Even more preferably, the chimeric polypeptide has an amino acid sequence according to SEQ ID NO: 24.

In one embodiment of the invention, exposure of a fusing polypeptide at the surface of the peroxisome is accompanied by over-expression of a complementing fusing polypeptide at the acceptor membrane-structure of the cell, e.g. the plasma membrane or the Golgi complex. A "complementing fusing polypeptide" according to the invention is a polypeptide involved in facilitating the fusion of a donor membrane structure, e.g. a vesicle of the Golgi complex or the ER, with an acceptor membrane-structure, e.g. the plasma membrane or the Golgi complex

A complementing fusing polypeptide is preferably a target-SNARE or t-SNARE (Jahn et al., Annu. Rev. Biochem. 863-911 (1999)). Preferred t-SNARE polypeptides are for instance Sso1 or Sso2, or homologues thereof, which are located at the plasma membrane, or Sed5, or homologues thereof, which is located at the Golgi complex (Burri et al. *supra*). Other preferred complementing fusing polypeptides are Sec9 involved in fusion at the plasma membrane or Bos1, Gos1 or Bet1 involved is fusion at the Golgi complex (Burri et al. *supra*), or homologues thereof.

According to a preferred embodiment, the fusing and complementing fusing polypeptides are exposed in stoichiometric amounts, meaning that the polypeptide interaction between a fusing and a complementing fusing polypeptide(s) is as close as possible to the physiological ratio or native stochiometry (Burri L et al, supra). A stoichiometric co-exposure is expected to further facilitate the fusion of the peroxisome with the acceptor membrane-structure, e.g. the plasma membrane. This stoichiometric co-expression is preferably achieved by using identical expression cassettes in substantially the same copy numbers.

According to one embodiment of the invention, the eukaryotic cell contains peroxisomes capable to fuse with the plasma membrane as well as the Golgi complex of the cell.

According to a preferred embodiment, all the chosen fusing and optional complementing fusing polypeptide(s), and the peroxisomal membrane polypeptide to be expressed are native to the eukaryotic host cell of choice.

The present invention further envisages improvements of eukaryotic cells according to the invention such that the eukaryotic cells are capable of fulfilling the activities according to the invention with an increased efficiency.

For instance, targeting of the peroxisome to the plasma membrane and subsequent membrane fusion may be enhanced by using the targeting mechanism of Golgi derived vesicles. Modification may allow this mechanism to be effective on peroxisomes.

An example to modify this mechanism to be effective on peroxisomes is to engineer Sec4, or a homologue thereof, such that it is operatively associated with the peroxisome. This will result in targeting of the peroxisome to the secretion complex in the plasma membrane (exocyst) and in addition will increase SNAREpin formation to enhance efficiency of peroxisomal fusion with the plasma membrane. Normally, Sec4 cycles between a GDP soluble state and a GTP bound secretion vesicle membrane attached state. It was demonstrated (Ossig et al 1995. EMBO Journal 3645-3653.) that a permanently attached Sec4 is biologically active. The membrane attachment of Sec4 is normally achieved by geranylgeranylation of the two C-terminal cysteine residues. To allow permanent attachment of Sec4 to the peroxisomal membrane, the two C-terminal cysteine residues of Sec4 may be deleted or substituted by a different amino acid and a chimeric polypeptide comprising a peroxisomal membrane polypeptide or part thereof, may be engineered analogous to the already described chimeric polypeptide comprising a fusing polypeptide and a peroxisomal membrane polypeptide.

In addition, the fusion of the peroxisome with the membrane-structure of the cell involved in the secretory pathway of the cell may be improved by making the complementing fusion polypeptide(s) more susceptible for interaction with the fusing polypeptide. This can be done in various ways, as described below.

A constitutively active mutant of a fusing polypeptide and, optionally, a complementing fusing polypeptide may be prepared and (over-)exposed at the surface of the peroxisome and, optionally, the acceptor membrane-structure, e.g. the plasma membrane. An example of a constitutively active mutant is a dephosphorylated mutant of a complementing fusing polypeptide, preferably a t-SNARE, more preferably Sso1 or a homologue thereof (Marash M, Gerst JE. t-SNARE dephosphorylation promotes SNARE assembly and exocytosis in yeast. EMBO J. 2001 Feb 1;20(3):411-21).

It is also possible to inactivate a regulator gene of a SNARE by techniques known to the skilled person. Such a regulator for instance is the v-SNARE master (VSM-1 or a homologue thereof), which binds to phosphorylated Sso1 and stabilizes the closed, inactive conformation of Sso1 (Marash M, Gerst JE. Mol Biol Cell. 2003 Aug;14(8):3114-25).

It is also possible to over-express an enzyme known to activate SNARE interactions. Preferably, the Ceramide Activated Phosphatase Protein (CAPP) or a homologue thereof is over-expressed (Fishbein JD, Dobrowsky RT, Bielawska A, Garrett S, Hannun YA. Ceramide-mediated growth inhibition and CAPP are conserved in Saccharomyces cerevisiae. J Biol Chem. 1993 May 5;268(13):9255-61).

It is also possible to modify the eukaryotic cell in such a way that the homeostasis of peroxisomes is affected. Such modification may comprise improving the rate of peroxisome biogenesis and/or decreasing the rate of peroxisome degradation as compared to the peroxisome biogenesis and/or degradation of the parental cell from which the eukaryotic cell according to the invention originates.

According to a more preferred embodiment, the eukaryotic cell has been genetically modified by (as described in WO 00/71579):
(a) over-expressing genes involved in peroxisome biogenesis, e.g. pex11 and/or pex3 or homologues thereof, and/or
(b) down-regulating the expression of genes involved in peroxisome degradation, e.g. vps15 and/or pdd1 and/or APG or homologues thereof..

The role of the pex3 and pex11 polypeptides in peroxisome biogenesis has already been described (Baerends RJ, et al Yeast. 1997 Dec;13(15):1449-63 and WO 00/71579). The role of Apg and Vps15 polypeptides in degradation of peroxisomes has also been described (Wang CW et al. J Biol Chem. 2001 Aug 10;276(32):30442-51 and Hutchins MU, et al. J Cell Sci. 1999 Nov;112 (Pt 22):4079-87).

The choice of a host cell to be modified according to the present invention will to a large extent depend upon the source of the nucleic acid sequence encoding a polypeptide of interest. Preferably, the eukaryotic cell is a mammalian, insect, plant, fungal, or algal cell. More preferably, the eukaryotic cell is a fungal cell, i.e. a yeast cell, such as *K. lactis* or S. cerevisiae or *Hansenula polymorpha* or *Pichia pastoris,* or a filamentous fungal cell. According to a most preferred embodiment, the eukaryotic cell is a filamentous fungal cell.

"Filamentous fungi" include all filamentous forms of the subdivision Eumycota and Oomycota (as defined by Hawksworth etal., 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* and *Trichoderma.*

Preferred filamentous fungal cells belong to a species of an *Aspergillus, Penicillium* or *Trichoderma* genus, most preferably a species of *Aspergillus niger, Aspergillus oryzae, Trichoderma reesei* or *Penicillium chrysogenum.*

### Polypeptides

In a second aspect, the present invention relates to novel polypeptides to be used for preparation of the eukaryotic cells of the first aspect. In particular, the present invention provides fusing polypeptides, chimeric polypeptides wherein a fusing polypeptide is operatively associated with a peroxisomal membrane polypeptide, peroxisomal membrane polypeptides and complementing fusing polypeptides, as defined herein above.

In one embodiment, the present invention provides a polypeptide displaying a v-SNARE function selected from the group consisting of (a) a polypeptide having an amino acid sequence according to SEQ ID NO: 13; (b) a polypeptide having an amino acid sequence that displays a degree of identity of at least 85%, preferably at least 90%, more preferably at least 93%, even more preferably at least 95% even more preferably at least 97%, even more preferably at least 98%, even more preferably at least 99% to the amino acid sequence according to SEQ ID NO: 13; and (c) a functional fragment of the polypeptide defined in (a) or (b).

In another embodiment, the present invention provides a peroxisomal membrane polypeptide selected from the group consisting of (a) a polypeptide having an amino acid sequence according to SEQ ID NO: 16; (b) a polypeptide having an amino acid sequence that displays a degree of identity of at least 85%, preferably at least 90%, more preferably at least 93%, even more preferably at least 95% even more preferably at least 97%, even more preferably at least 98%, even more preferably at least 99% to the amino acid sequence according to SEQ ID NO: 16; and (c) a functional fragment of the polypeptide defined in (a) or (b)..

In still another embodiment, the present invention provides a chimeric polypeptide suitable to obtain exposure at the surface of a peroxisome of an amino acid sequence corresponding to the amino acid sequence of a fusing polypeptide exposed at the surface of a donor membrane-structure, wherein the chimeric polypetide comprises a fusing polypeptide or part thereof operatively associated with a peroxisomal membrane polypeptide or a part thereof.

Preferably, the fusing polypeptide component of the chimeric polypeptide comprises an amino acid sequence selected from the group consisting of (a) a sequence corresponding to position 1 to 95 of SEQ ID NO:13 and (b) a homologous sequence displaying a degree of identity of at least 50%, preferably at least 60%, more preferably at least 70%, even more preferably at least 80%, most preferably at least 90% to the sequence defined in (a).

Also preferably, the peroxisomal membrane protein component of the chimeric polypeptide comprises an amino acid sequence selected from the group consisting of (a) a sequence corresponding to position 2 to 224 of SEQ ID NO:16, preferably corresponding to a position 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 to position 224 and (b) a homologous sequence displaying a degree of identity of at least 50% preferably at least 60%, more preferably at least 70%, even more preferably at least 80%, most preferably at least 90% to the sequence defined in (a).

More preferably, the chimeric polypeptide has an amino acid sequence according to SEQ ID NO: 24.

For the purpose of the present invention, the degree of identity between two amino acid sequences refers to the percentage of amino acids that are identical between the two sequences. First, homologous polypeptide sequences are searched using the Basic Local Alignment Search Tool (BLAST) algorithm, which is described in Altschul, et al., J. Mol. Biol. 215: 403-410 (1990). Software for performing BLAST analyses is publicly available through the National Center for Biotechnology Information (http://www.ncbi.nlm.nih.gov/). The BLAST algorithm parameters W, B, and E determine the sensitivity and speed of the alignment. The BLAST program uses as defaults a word length (W) of 3, the BLOSUM62 scoring matrix (see Henikoff & Henikoff, Proc. Natl. Acad. Sci. USA 89: 10915 (1989)) alignments (B) of 50, expectation (E) of 10, M=5, N=-4.

Next, the degree of identity (as defined above) of homologous sequences is determined using the CLUSTALW alignment algorithm (Higgins D. et al (1994). Nucleic Acids Res. 22:4673-4680) using the following parameters; Gap size: 5, Gap open: 11, Gap extension: 1, Mismatch: -15, Word size: 3.

### Polynucleotides

In a third aspect, the present invention relates to polynucleotides comprising nucleic acid sequences encoding any of the polypeptides of the second aspect.

The invention encompasses nucleic acid sequences encoding fusing polypeptides, chimeric polypeptides wherein a fusing polypeptide is operatively associated with a peroxisomal membrane polypeptide, peroxisomal membrane polypeptides and complementing fusing polypeptides, as defined herein.

After having chosen a eukaryotic cell to be modified according to the invention, the identity (source) of the fusing polypeptide, the polypeptide functioning as the peroxisomal membrane anchor and, optionally, the complementing fusing polypeptide may be established. For instance, the source of nucleic acid sequences encoding the polypeptides of the second aspect may depend on the identity of the eukaryotic cell of choice. Preferably, the nucleic acid sequences encoding the polypeptides of the second aspect are endogenous to the eukaryotic cell to be modified according to the invention.

The present invention further provides a nucleic acid construct comprising a nucleic acid sequence encoding a polypeptides as defined above, operatively associated with one or more control sequences directing the expression of the polypeptide in a suitable host.

Expression will be understood to include any step involved in the production of the polypeptide including, but not limited to, transcription, post-transcriptional modification, translation, post-translational modification.

"Nucleic acid construct " is defined herein as a nucleic acid molecule, either single-or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette or vector when the nucleic acid construct contains all the control sequences required for expression of a coding sequence.

The term "control sequences" is defined herein to include all components, which are necessary or advantageous for the expression of a polypeptide in general. Each control sequence may be native or foreign to the nucleic acid sequence encoding the polypeptide. Such control sequences include, but are not limited to, a promoter, a leader, optimal translation initiation sequences (as described in Kozak, 1991, J. Biol. Chem. 266:19867-19870), a polyadenylation sequence, a transcription terminator. At a minimum, the control sequences include a promoter, and transcriptional and translational stop signals.

The term "operatively associated" is defined herein as a configuration in which a control sequence is appropriately placed at a position relative to the coding sequence of the DNA sequence such that the control sequence directs the expression of a polypeptide.

The control sequence may be any appropriate promoter sequence, which shows transcriptional activity in the cell, including mutant, truncated, and hybrid promoters, and may be obtained from genes encoding extra-cellular or intracellular polypeptides either homologous (native) or heterologous (foreign) to the cell.

The promoter may be the promoter natively associated with the coding sequence to be expressed. The promoter may also be a constitutive or inducible promoter foreign to the coding sequence to be expressed.

Examples of preferred inducible promoters that can be used are a starch-, copper-, oleic acid- inducible promoter.

According to another preferred embodiment, if a gene has to be over-expressed in the host cell of the invention, such as CAPP, a strong inducible promoter is used such as the glucoamylase promoter of *A. niger* or the TAKA amylase promoter of *A. oryzae.*

The control sequence may also be a suitable transcription terminator sequence, a sequence recognized by cell to terminate transcription. The terminator sequence is operably linked to the 3' terminus of the nucleic acid sequence encoding the polypeptide. Any terminator, which is functional in the cell, may be used in the present invention.

The nucleic acid construct may be identical to or cloned in a vector or expression vector.

The recombinant expression vector may be any vector (e.g., a plasmid or virus), which can be conveniently subjected to recombinant DNA procedures and can bring about the expression of the nucleic acid sequence encoding the fusing polypeptide (or complementing fusing polypeptide). The choice of the vector will typically depend on the compatibility of the vector with the eukaryotic cell into which the vector is to be introduced. The vectors may be linear or closed circular plasmids. The vector may be an autonomously replicating vector, i. e., a vector, which exists as an extrachromosomal entity, the replication of which is independent of chromosomal replication, e.g., a plasmid, an extrachromosomal element, a minichromosome, or an artificial chromosome. An autonomously maintained cloning vector, which may be used in filamentous fungal cells comprise the AMA1-sequence (see e.g. Aleksenko and Clutterbuck (1997), Fungal Genet. Biol. 21: 373-397).

Alternatively, the vector may be one which, when introduced into the cell, is integrated into the genome and replicated together with the chromosome (s) into which it has been integrated. The integrative vector may integrate at random or at a predetermined target locus in the chromosomes of the host cell.

In a preferred embodiment of the invention, the integrative vector comprises a DNA fragment, which is homologous to a DNA sequence in a predetermined target locus in the genome of the host cell for targeting the integration of the vector to this predetermined locus. In order to promote targeted integration, the vector is preferably linearized prior to transformation of the host cell. Linearization is preferably performed such that at least one but preferably either end of the cloning vector is flanked by sequences homologous to the target locus. The length of the homologous sequences flanking the target locus is depending on the identity of the host cell. For a fungus, the length is preferably at least 0.1kb, even preferably at least 0.2kb, more preferably at least 0.5 kb, even more preferably at least 1 kb, most preferably at least 2 kb. Preferably, the DNA sequence in the vector, which is homologous to the target locus is derived from a highly expressed locus meaning that it is derived from a gene, which is capable of high expression level in the host cell. A gene capable of high expression level, i.e. a highly expressed gene, is herein defined as a gene whose mRNA can make up at least 0.5% (w/w) of the total cellular mRNA, e.g. under induced conditions, or alternatively, a gene whose gene product can make up at least 1% (w/w) of the total cellular protein (as described in EP 357 127). A number of preferred highly expressed fungal genes are given by way of example: the amylase, glucoamylase, alcohol dehydrogenase, xylanase, glyceraldehyde-phosphate dehydrogenase or cellobiohydrolase genes from *Aspergilli* or *Trichoderma.* Most preferred highly expressed genes for these purposes are a glucoamylase gene, preferably an *A. niger* glucoamylase gene, an *A. oryzae* TAKA-amylase gene, an *A. nidulans gpdA* gene or a *Trichoderma reesei* cellobiohydrolase gene. This type of expression vector is highly suited to over-express a given gene in the eukaryotic cell of the invention, such as CAPP or a wild type or constitutively active mutant of a fusing polypeptide or a complementing fusing polypeptide (sso1).

If a gene of the host has to be inactivated such as VSM1, it is preferably performed by designing an inactivation vector and targeting the vector at the locus of the gene to be inactivated according to the techniques described in EP 635 574.

The vector system may be a single vector or two or more vectors, which together contain the total DNA to be introduced into the genome of the cell. The vectors preferably contain one or more selectable markers, which permit easy selection of transformed cells. A selectable marker is a gene the product of which provides for biocide or viral resistance, resistance to heavy metals, prototrophy to auxotrophs, and the like.

The introduction of an expression vector or a nucleic acid construct into a cell is done using commonly known techniques. It may involve a process consisting of protoplast formation, transformation of the protoplasts, and regeneration of the cell wall in a manner known per se. Selected transformed cells are then analysed for the capacity of peroxisomes to fuse with a membrane-structure involved in the secretory pathway of the cell, e.g the plasma membrane.

Several methods are available to analyse whether cells have obtained the capacity of fusion of the peroxisome with a membrane-structure of the cell involved in the secretory pathway of the cell.

According to one embodiment, the morphology of the cells is studied under electronic or fluorescence microscopy, using peroxisomal-specific labelling to visualize the fusion of the peroxisome with a membrane-structure of the cell involved in the secretory pathway of the cell. Examples of peroxisomal-specific labelling are:
- thiolase labelling as described by : Simon M, Binder M, Adam G, Hartig A, Ruis H Control of peroxisome proliferation in Saccharomyces cerevisiae by ADR1, SNF1 (CAT1, CCR1) and SNF4 (CAT3). Yeast. 1992 Apr;8(4):303-9, or
- GFP labelling as described by Monosov EZ, Wenzel TJ, Luers GH, Heyman JA, Subramani S Labeling of peroxisomes with green fluorescent protein in living P. pastoris cells. J Histochem Cytochem. 1996 Jun;44(6):581-9, or
- catalase labelling as described by Kunce CM, Trelease RN, Turley RB. Purification and biosynthesis of cottonseed (Gossypium hirsutum L.) catalase. Biochem J. 1988 Apr 1;251(1):147-55.

According to another embodiment, the fusion of the peroxisome with a membrane-structure of the cell involved in the secretory pathway of the cell is monitored by expressing a model polypeptide in the peroxisome of the cell, preferably using a signal promoting peroxisome localisation of the model polypeptideand measuring the presence of the model polypeptide in the fermentation broth. A preferred model polypeptide is Green Fluorescent Protein (GFP), since its presence in the fermentation broth can be visualised by fluorescence and monitored by western blotting. Other model polypeptides may be enzymatically active intracellular proteins operatively associated with a peroxisomal localization signal, like acetamidase, or naturally peroxisomal localised proteins like catalase, amadoriase or thiolase.

Alternatively to or in combination with methods described above a eukaryotic cell containing peroxisomes that are capable to fuse with a membrane-structure of the cell involved in the secretory pathway of the cell is obtained by classical strain improvement, using an appropriate screening method. A preferred screening method uses expression of a model polypeptide like GFP as described above.

Preferably, the eukaryotic cell containing peroxisomes that are capable to fuse with a membrane-structure of the cell involved in the secretory pathway displays a peroxisomal fusion efficiency to an extent that at least 10% of the total amount of produced polypeptide is secreted into the culture medium at a given time point during cultivation, more preferably at least 40% of the produced polypeptide is secreted, even more preferably at least 60% of the produced polypeptide is secreted, even more preferably at least 70% of the produced polypeptide is secreted, even more preferably at least 80% of the produced polypeptide is secreted, and most preferably at least 90% of the produced polypeptide is secreted. The total amount of produced polypeptide is defined as the amount of polypeptide present in the fermentation broth, where the fermentation broth is defined to consist of a biomass fraction and a medium fraction. The amount of secreted polypeptide may be estimated using a model polypeptide. This model polypeptidemay be Green Fluorescent Protein (GFP) with an engineered PTS-1 (e.g. GFP-SKL). The concentration of GFP-SKL within the fermentation broth fractions can be determined using techniques known the person skilled in the art (e.g. fluorescence measurement, absorbance measurement, Western blot). Using the determined concentrations of the model polypeptide, the fraction of secreted model polypeptide can be calculated as percentage of the total amount of produced model polypeptide.

### Production of a polypeptide of interest

In a further aspect, the present invention relates to the production of a polypeptide of interest using the eukaryotic cell of the first aspect as host cell.

To that end, the eukaryotic cell of the first aspect additionally comprises a nucleic acid construct or an expression vector comprising a nucleic acid sequence encoding a polypeptide of interest and operatively associated therewith a nucleic acid sequence encoding a signal that promotes peroxisomal localisation of the polypeptide of interest.

The signal promoting peroxisomal localisation can be any signal as long as it allows the localisation and/or accumulation of the associated polypeptide inside the peroxisome.

Preferably, the signal promoting peroxisomal localisation is selected from the group consisting of:
(a) a tripeptide wherein the first amino acid in the N- to C-terminal direction is A, C, H, K, N, P, S or T, the second amino acid in the N- to C-terminal direction is H, K, N, Q, R or S and the third amino acid in the N- to C-terminal direction is A, F, I, L, M or V, and
(b) a peptide defined as follows: (R/K) (L/V/I/Q) XX (L/V/I/H/Q) (US/G/A/K) X(H/Q)(L/A/F), wherein X may be any amino acid.

More preferably, the tripeptide defined in (a), also named PTS-1, is present as a C-terminal extension of the polypeptide to be produced in the peroxisome. Thus, the DNA sequence coding for the signal promoting peroxisomal localisation is cloned downstream of and in operative association with the DNA sequence encoding the polypeptide.

According to a preferred embodiment, the tripeptide defined in (a) is a variant of [PAS]-[HKR]-[L] as described in: *In silico* prediction of the peroxisomal proteome in fungi, plants and animals. Olof Emanuelsson, Arne Elofsson, Gunnar von Heijne and Susana Cristo' bal. J. Mol. Biol. (2003) 330, 443-456. According to a more preferred embodiment, the tripeptide defined in (a) is SKL or PRL.

According to another preferred embodiment, the tripeptide defined in (a) has a sequence that allows removal of the tripeptide sequence or removal of the tripeptide sequence together with a sequence preceding the tripeptide sequence from the C-terminus of the polypeptide of interest once the polypeptide is secreted outside the cell, by for instance action of a suitable protease or peptidase.

Peptides defined in (b) are also named PTS2 signals (Swinkels, B et al 1991. EMBO Journal 3255-3262; Petriv O.I. et al 2004. The Journal of molecular Biology 119-134). Preferably, they are present in the N-terminal part of the polypeptide.

If the polypeptide of interest already contains a signal promoting peroxisomal localisation, preferably this native signal is used to promote peroxisomal localisation. Alternatively, one may choose to replace the native signal promoting peroxisomal localisation by a different one.

In one embodiment, the present invention envisages a phased extra-cellular production of the polypeptide of interest present in the peroxisome. In a first phase the polypeptide of interest accumulates in the peroxisome and in a second phase the inducible promoter driving the expression of the fusing polypeptide (optionally complementing fusing polypeptide) is induced by adding a specific inducer to the medium, which will in turn lead to the fusion of the peroxisome with an acceptor membrane structure of the cell involved in the secretory pathway of the cell. This will result in the extra-cellular production of the polypeptide of interest.

Alternatively, other types of phased production are possible: first the production of the polypeptide of interest, then induction of peroxisome proliferation and as a last step induction of the fusion of the peroxisome with an acceptor membrane structure of the cell involved in the secretory pathway of the cell.

The polypeptide may be any polypeptide native or foreign to the host cell. The term "foreign polypeptide" is defined herein as a polypeptide, which is not naturally produced by a given cell. The term "polypeptide" is not meant herein to refer to a specific length of the encoded polypeptide produced and therefore encompasses peptides, oligopeptides and proteins.

The host cell of the invention is highly suited for the production of polypeptides needing a reducing environment for maturation, e.g. intracellular polypeptides. Thus, according to a preferred embodiment, the polypeptide of interest is an intracellular polypeptide. Accordingly, the method of the invention using the host cell of the invention is the first one able to produce intracellular polypeptide in the fermentation medium on an industrial scale.

A preferred polypeptide is an enzyme naturally produced in the peroxisome, such as amadoriase, catalase, acyl-CoA oxidase, linoleate isomerase, trans-2-enoyl-ACP reductase, trichothecene 3-O-acetyltransferase, alcohol dehydrogenase, carnitine racemase, D-mandelate dehydrogenase, enoyl CoA hydratase, fructosyl amine oxygen oxidoreductase, 2-hydroxyhepta-2,4-diene-1,7-dioate isomerase, NADP-dependent malate dehydrogenase, oxidoreductase, quinone reductase All these enzymes contain a C-terminal SKL sequence.

Other intracellular enzymes are ceramidases, epoxide hydrolases aminopeptidases, acylases, aldolase, hydroxylase, aminopeptidases.

In another embodiment, the polypeptide is an antibody or portion thereof, an antigen, a clotting factor, an extracellular enzyme, a hormone or a hormone variant, a receptor or portions thereof, a regulatory protein, a structural protein, a reporter, or a transport protein. The present invention thereby advantageously allows the extracellular production of polypeptides that encounter difficulties in the normal secretory pathway of the cell.

The extracellular enzyme may be an aminopeptidase, amylase, carbohydrase, carboxypeptidase, catalase, cellulase, chitinase, cutinase, deoxyribonuclease, dextranase, esterase, alpha-galactosidase, beta-galactosidase, glucoamylase, alpha-glucosidase, beta-glucosidase, haloperoxidase, hydrolase, invertase, isomerase, laccase, ligase, lipase, lyase, mannosidase, mutanase, oxidase, oxidoreductase pectinase, peroxidase, phytase, polyphenoloxidase, protease, ribonuclease, transferase, transglutaminase, or xylanase. The nucleic acid sequence encoding a polypeptide of interest may be obtained from any prokaryotic, eukaryotic, or other source. For purposes of the present invention, the term "obtained from "as used herein in connection with a given source shall mean that the polypeptide is produced by the source or by a cell in which a gene from the source has been inserted.

The host cells of the present invention are cultivated in a nutrient medium suitable for production of the polypeptide of interest using methods known in the art. For example, the cells may be cultivated by shake flask cultivation, small-scale or large-scale fermentation (including continuous, batch, fedbatch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art (see, e. g., Bennett,J. W. and LaSure, L.,eds., More Gene Manipulations in Fungi, Academic Press, CA, 1991). Suitable media are available from commercial suppliers or may be prepared using published compositions (e. g., in catalogues of the American Type Culture Collection).

The cultivation medium may be adapted to the polypeptide to be produced and to the eukaryotic cell chosen. According to a preferred embodiment, the cultivation medium comprises an activator of Ceramide Activated Phosphatase Protein (CAPP). This phosphatase is known to activate SNARE interactions by dephosphorylating t-SNARE's. (Marash M, Gerst JE :t-SNARE dephosphorylation promotes SNARE assembly and exocytosis in yeast. EMBO J. 2001 Feb 1;20(3):411-21). An example of an activator of CAPP is a ceramide, e.g. dihydro-C₂ ceramide or another C2-ceramide (Calbiochem, SIGMA). Preferably, 1 to 100 µM ceramide is present in the fermentation medium at the beginning of the fermentation. More preferably, 5 to 50µM ceramide is present in the fermentation medium at the beginning of the fermentation. Even more preferably, about 10µM ceramide is present in the fermentation medium at the beginning of the fermentation. According to a most preferred embodiment, the concentration of ceramide is monitored during the whole fermentation process to be kept at such values. If needed, fresh ceramide may be continuously added during the fermentation process.

According to another preferred embodiment, the fermentation medium comprises a substance inducing peroxisome proliferation, such as a substrate that is metabolised via at least one enzyme that is normally located in the peroxisome, preferably a fatty acid, more preferably oleate, as previously described (YASUYOSHI SAKAI et al, Regulation of Peroxisomal Proteins and Organelle Proliferation by Multiple Carbon Sources in the Methylotrophic Yeast, Candida boidinii. Yeast 14, 1175-1187 (1998)). Fatty acids can also be used to obtain peroxisomal proliferation as previously described (Intrasuksri U, et al, Mechanisms of peroxisome proliferation by perfluorooctanoic acid and endogenous fatty acids. Gen Pharmacol. 1998 Aug;31 (2):187-97.) These two references are incorporated by reference in this context to define the amount of peroxisome-inducing substances needed.

According to a more preferred embodiment, the fermentation medium comprises both an activator of CAPP and a peroxisome-inducing substance.

The resulting polypeptide may be isolated from the culture broth by methods known in the art. For example, the polypeptide may be isolated from the culture broth by conventional procedures including, but not limited to, centrifugation, filtration, extraction, spray drying, evaporation, or precipitation. The isolated polypeptide may then be further purified by a variety of procedures known in the art including, but not limited to, chromatography (e. g., ion exchange, affinity, hydrophobic, chromatofocusing, and size exclusion), electrophoretic procedures (e.g., preparative isoelectric focusing, differential solubility (e. g., ammonium sulfate precipitation), or extraction (see, e.g., Protein Purification, J.-C. Janson and Lars Ryden, editors, VCH Publishers, New York, 1989). The method of the invention advantageously allows the production of at least 0.01 g/I of the polypeptide of interest at the end of the fermentation period. Preferably at least 0.05 g/l of the polypeptide is produced, more preferably at least 0.1g/l, even more preferably at least 0.5g/l and most preferably at least 1 g/l.

All patents and publications, including all sequences and methods disclosed within such patents and publications, referred to herein are expressly incorporated by reference. These patents and publications include: EP 357 127, EP 635 574, EP 238 023, WO 97/06261, WO 98/46772, WO 00/71579.

The present invention is further described by the following examples, which should not be construed as limiting the scope of the invention.

### Example 1

### Example 1: Targeting an acetamidase (amdS) protein in peroxisome in Aspergillus niger and Kluyveromyces lactis using a C-terminal SKL tag

The *Aspergillus niger* strain (CBS 513.88) and *K.lactis* strain (CBS 685.97) used were already deposited. In these strains, using classical molecular biology techniques, as described in Molecular Cloning: A Laboratoy Manual, Sambrook *et al.,* New York: Cold Spring Harbour Press, 1989, several genes were over-expressed and activity of the encoded proteins was determined as described below.

### 1.1. Cloning the A. niger acetamidase gene with and without SKL tag and expression in A. niger and K.lactis

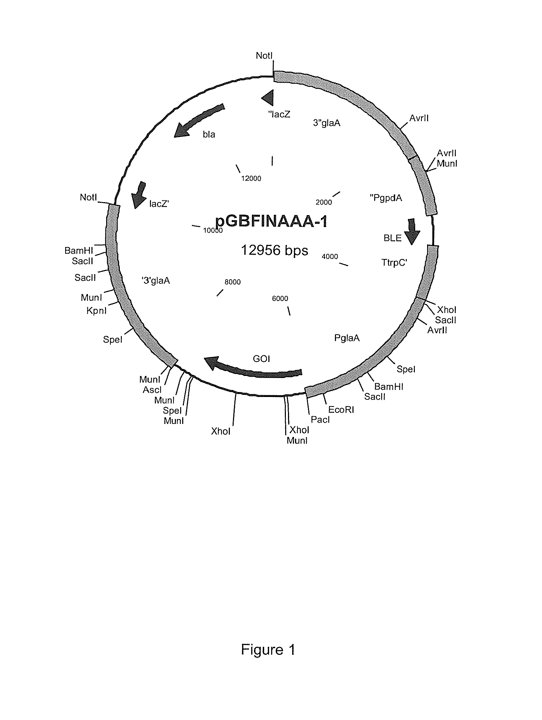

Genomic DNA from CBS 513.88 was used as template in a PCR reaction using SEQ ID NO: 1 and SEQ ID NO: 2, resulting in coding sequence shown as SEQ ID NO: 4. In addition genomic DNA from CBS 513.88 was used as template in a PCR reaction using SEQ ID NO: 1 and SEQ ID NO: 3, resulting in coding sequence shown as SEQ ID NO: 5. All PCR reactions, cDNA synthesis, ligations and transformations were performed as described above in Molecular Cloning. The resulting PCR fragments (SEQ ID NO: 4 and SEQ ID NO: 5) were cut with Pacl and Ascl according to the manufacturers instructions and ligated in a Pacl, Ascl linearized *A. niger* expression vector depicted in figure 1, resulting in a construct in which the acetamidase gene is placed under control of the glaA promoter. This expression vector was used to transform *A. niger.*

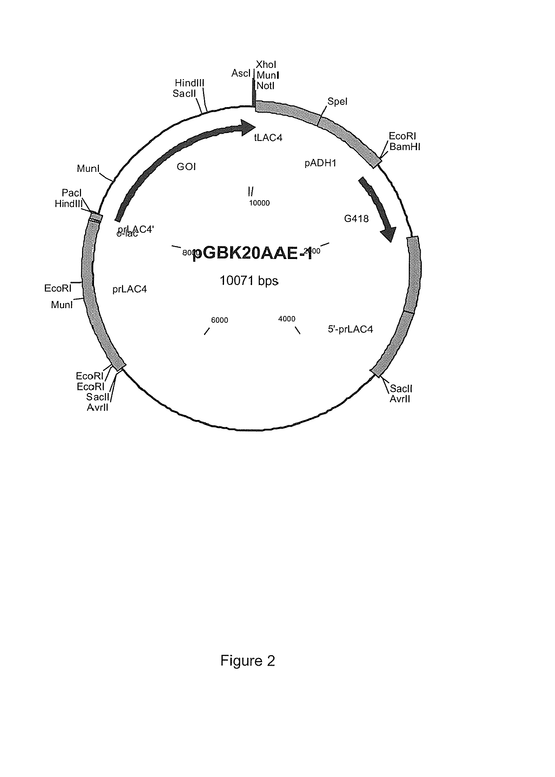

In addition, genomic DNA from CBS 513.88 was used as template in a PCR reaction using SEQ ID NO: 6 and SEQ ID NO: 7, resulting in coding sequence shown as SE ID NO: 9. Furthermore, genomic DNA from CBS 513.88 was used as template in a PCR reaction using SEQ ID NO: 6 and SEQ ID NO: 8, resulting in coding sequence shown as SEQ ID NO: 10. The resulting PCR fragments (SEQ ID NO: 9 and SEQ ID NO: 10) were cut with Pacl and Ascl according to the manufacturers instructions and ligated in a Pacl, Ascl linearized *K.lactis* expression vector depicted in figure 2, resulting in a construct in which, the *A. niger* acetamidase gene (SEQ ID NO: 9 and SEQ ID NO: 10) is placed under control of the lac4 promotor. This expression vector was used to transform *K. lactis.*

The resulting plasmids were transformed respectively to *A. niger* CBS 513.88 or to *K. lactis* CBS 685.97. The transformation of A niger was performed according to (Kelly JM, Hynes MJ Transformation of Aspergillus niger by the amdS gene of Aspergillus nidulans. EMBO J. 1985 Feb;4(2):475-9) and of K lactis according to (Sreekrishna K, Webster TD, Dickson RC, Transformation of Kluyveromyces lactis with the kanamycin (G418) resistance gene of Tn903. Gene. 1984 Apr;28(1):73-81).

### 1.2.Cultivation of the A. niger and K. lactis transformants and determination of the intracellular acetamidase activity

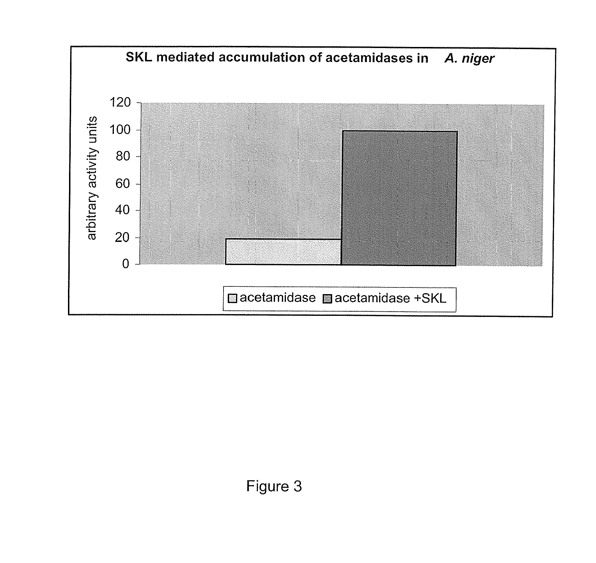

The presence of the expression cassette in the *A. niger* transformants was checked by PCR. The selected transformants were cultivated for 5 days at 30 °C at 250 rpm in 500 ml conical shake flasks with baffles in 100 ml of the following medium: 150 g/l maltose, 60 g/l bacto soytone, 1 g/l NaH₂PO₄, 15 g/l (NH₄)₂SO₄, 1 g/l MgSO₄.4H₂O, 0.08 g/l tween-80, 0.02 g/l Basildon, 20 g/l Morpholino Ethane Sulfonicacid (MES), 1 g/l L-arginine. Cells were harvested and disrupted by grinding under liquid nitrogen. Cell lysates were obtained by resuspending 25 mg grinded biomass in 0.5 ml phosphate buffered D₂O (deuterium pD 7.0). Subsequently 0.5 ml of 10 mg/ml substrate (propionamide) in D₂O was added and incubated at 37ºC for 4 days and centrifuged (end concentration 5 mg/mL substrate and 25 mg/mL extract).Measurement of the acetamidase activity in the control cell CBS513.88 and in the transformed cell was performed by Nuclear Magnetic Resonance as described by the manufacturer's instructions (figure 3). ¹H NMR spectra were recorded on a Bruker DRX-600 operating at a proton frequency of 600 MHz at a probe temperature of 300 K. A 5mm triple resonance probe with self-shielded gradients was used. ¹H NMR spectra of all reference compounds were acquired in order to show that all the compounds involved have unique NMR signals, based on which they can be identified and quantified (not shown). In order to create perfect reference spectra of every relevant compound, a stock solution of each compound was prepared in D₂O (Cambridge Isotope Laboratories). Stock solutions were prepared in concentrations of 10 mg/mL by weighting the substrate or the reference compound and adding D₂O. From these stock solutions 500 µL was mixed with 500 µL 0.5 M phosphate buffer pH 6.96 (KH₂PO₄/K₂HPO₄). ¹H-NMR spectra of each compound, i.e. acrylamide, acrylic acid, acetamide, acetic acid, propionamide and propionic acid were subsequently collected at 600 MHz in D₂O at 27ºC (endconcentration 5 mg/mL substrate or reference compound in D₂O). Unique chemical shifts, which did not overlap with signals caused by other compounds, were identified for each compound. In addition, the purity of each reference was checked and the absence of possible contaminants was confirmed.

The compounds had the following characteristic signals:
- Acrylamide (catalog number 8.00830, lot 4202056, Merck NJ USA): 5.82 (dd, Hb, J = 10.3 Hz, 1.2 Hz), 6.22 (dd, Hc, J = 17.2 Hz, 1.2 Hz), 6.28 (d, Ha, J = 10.3 Hz), 6.31 (d, Ha, J = 10.3 Hz) ppm.
- Acrylic acid (catalog number 14,723-0, lot S17163-034, Aldrich, WI USA): 5.65 (dd, Hb, J = 10.4 Hz, 1.6 Hz), 6.01 (dd, Hc, J = 17.4 Hz, 1.6 Hz), 6.11 (d, CH2=CH, 3J = 10.3 Hz), 6.14 (d, CH2=CH, 3J = 10.3 Hz).
- Acetamide (catalog number 12,263-7, lot16813BA-453, Aldrich, WI USA): 1.99 (s, CH3) ppm.
- Acetic acid (catalog number 1.00063, lot K31668363, Merck NJ USA): 1.90 (s, CH3) ppm.
- Propionamide (catalog number 14,393-6, lot 25009JB-413, Aldrich, WI USA): 1.10 (t, CH3), 2.27 (q, CH2) ppm.
- Propionic acid (catalog number P-1386, lot 083 K3404, Sigma, St Louis, Mo USA): 1.09 (t, CH3), 2.37 (q, CH2) ppm.

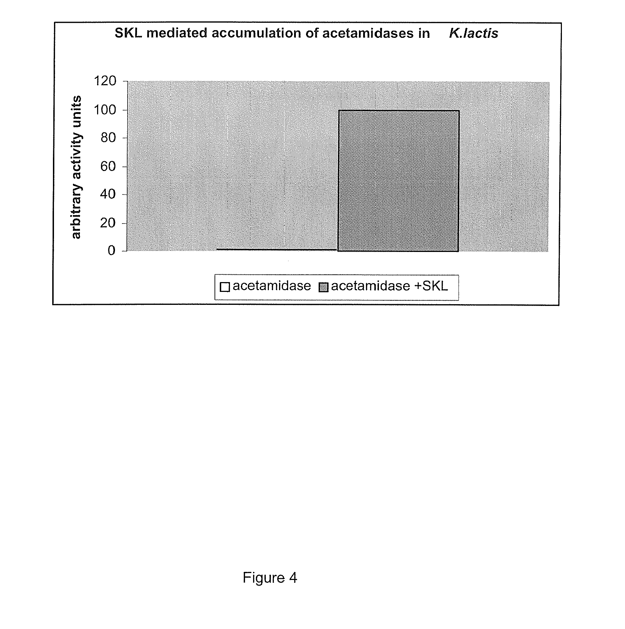

The resulting *K.lactis* transformants (checked by PCR) were cultivated for 3 days in 100 ml YEPD (Yeast Extract 10 g/l, Peptone 20 g/l, Dextrose 20 g/l) at 30 °C at 250 rpm in 500 ml conical shake flasks with baffles. Cells were harvested and disrupted by grinding under liquid nitrogen. Cell lysates were obtained by resuspending grinded biomass in 20 mM sodiumphosphatebuffer, pH 7.4, 1 mM EDTA, 2 mM DTT with protease inhibitor (complete from Roche: catalogus number 1873580). Acetamidase activities were determined in the control *K lactis* cell and in the transformed cell according to Skouloubris et al., Molecular Microbiology (2001) 40(3), 596-609 (figure 4).

As clearly shown in figures 3 and 4, the addition of the C-terminal SKL tag, which promotes peroxisomal localisation of the acetamidase protein, increases the intracellular acetamidase enzyme activity in *A. niger* and *K.lactis.*

### Example 2: Addition of oleate to the fermentation medium to increase peroxisome proliferation

An increase of the number of peroxisomes per cell and thus an increase in peroxisomal storage volume of *A. niger* was mediated by supplementing the culture medium with Na-oleate (catalogus number 26125, Riedel-de Haën, Hannover, Germany) and Tween-40 (catalogus number 93775, Fluka, Buchs, Switzerland). The increased number of peroxisomes was demonstrated by fluorescence microscopy using Green Fluorescent Protein (GFP)(Chalfie, M et al., Science (1994) 263(5148): 802-805) with an engineered C-terminal SKL tag. The C-terminal -SKL tag was engineered to the widely used and well-characterised GFP gene using PCR methodology as described in Example 1. The resulting GFP-SKL gene was cloned into an *A. niger* expression vector and transformed to *A*. *niger* CBS513.88 using methodology as described in Example 1.

The resulting *A*. *niger* strains over-expressing GFP with a C-terminal linked -SKL peptide were cultivated in shake flasks using an orbital shaker at 30ºC, 250 RPM in Minimal Enriched Aspergillus Medium (MEAM, described in patent application WO 98/46772). After 48 hours of pre-culture, mycelium was harvested and, washed and subsequently transferred to fresh culture medium (MEAM) supplemented with 0.18% (w/v) Na-oleate and 0.02% (w/v) Tween-40). Control cultures were cultured in MEAM without supplementation with Na-oleate and Tween-40. After 26 hours of culture, samples were analysed using a Zeiss fluorescence microscope, using blue light excitation (490nm). Representative samples were used for fluorescence photography using Fujicolor 800ASA colorfilm.

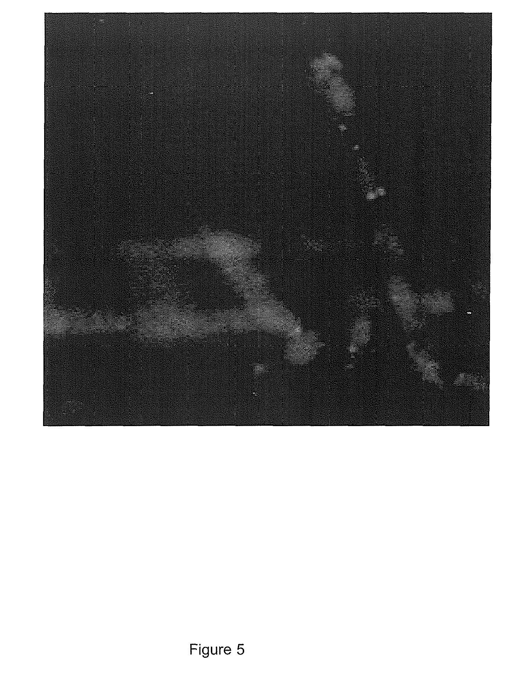

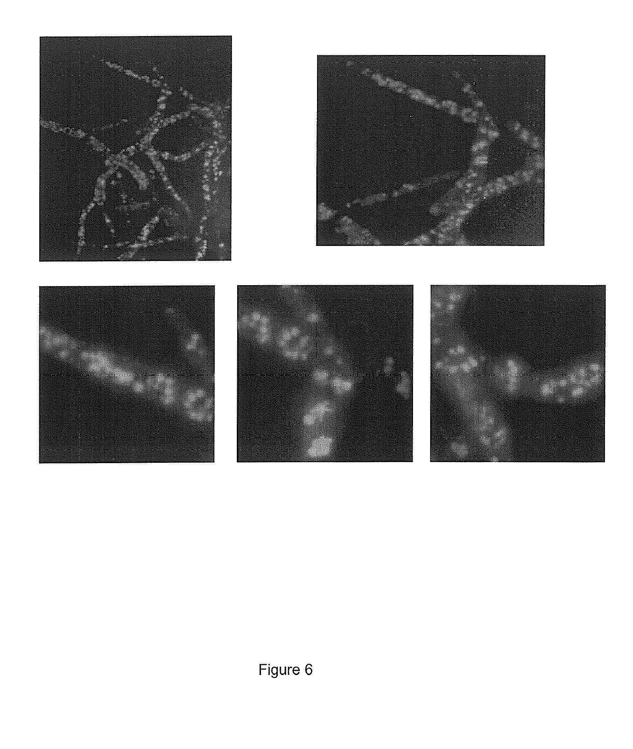

As can be observed in figure 5, cultivation of a GFP-SKL over-expressing strain, in a medium, which does not comprise Na-oleate and Tween-40, resulted in few peroxisomes per cell. As clearly demonstrated in figure 6, cultivating the same cell in a fermentation medium supplemented with Na-oleate and Tween-40, induced peroxisome proliferation and resulted in increased numbers of peroxisomes per cell.

### Example 3. Release of the peroxisomal content outside the cell by fusion of the peroxisome with the plasma membrane

### 3.1 Cloning and expression of a fusing polypeptide at the surface of a peroxisome

*A. niger* v-SNARE SncA, shown as SEQ ID NO's: 11, 12 and 13, genomic, cDNA and protein sequence, respectively, without transmembrane domain, was fused to peroxisomal membrane protein 22 (Pmp22), shown as SEQ ID NO's: 14, 15 and 16, genomic, cDNA and protein sequence, respectively. Genomic DNA from CBS513.88 was used as template in a PCR reaction using SEQ ID NO: 17 and SEQ ID NO: 18 to result in coding SEQ ID NO: 19. In addition, genomic DNA from CBS513.88 was used as template in a PCR reaction using SEQ ID NO: 20 and SEQ ID NO: 21 to result in coding SEQ IDNO: 22. Subsequently, SEQ ID NO: 19 and SEQ ID NO: 22 and were used as template in a PCR reaction using SEQ ID NO: 17 and SEQ ID NO: 21 to result in SEQ ID NO: 23. The resulting PCR fragment (SEQ ID NO: 23) was digested with restriction enzymes *Pac*l and *Asc*l according to the manufacturers instructions and ligated into a *Pac*l, *Asc*l linearised *A. niger* expression vector as depicted in figure 1. This resulted in a construct in which the gene encoding the fusing polypeptide with peroxisomal membrane anchor (SncA/Pmp-22) was placed under control of the glaA promoter. Expression of the gene resulted in a chimeric protein comprising a peroxisomal membrane anchor, as shown as SEQ ID NO: 24 The SncA/Pmp-22 expression vector together with a GFP-SKL expression vector was used to co-transform *A*. *niger.* All PCR reactions, ligations and transformations were performed using classical molecular biology techniques, as described in Molecular Cloning: A Laboratory Manual, Sambrook et al., New York: Cold Spring Harbor Press,1989 .

### 3.2 Cultivation of the co-transformants for analysis of release of peroxisomal content

Amongst several clones of *A. niger* strains over-expressing GFP-SKL and SncA/Pmp-22 as obtained in the former paragraph, a selection was made using PCR for clones containing a single copy of each gene. The clone selected was cultivated in shake flasks in an orbital shaker at 30ºC, 250 RPM in MEAM buffered medium (described extensively in patent application WO 98/46772) with and without 10 µM C2-ceramide (N-Acetyl-D-sphingosine: catalogues number A7191, Sigma, St. Louis Missouri USA). A GFP-SKL over-expressing strain containing a single gene copy (construction described in Example 2) and an empty host strain (CBS513.88) were used as controls. Samples were taken at 24 and 48-hour time points. The supernatant samples were cleared by centrifugation and separated from residual cell debris by ultrafiltration through .45µm filters (catalogus number 4614, Pall, Ann Arbor, Mo USA).

### 3.3 SDS-PAGE analysis of the release of peroxisomal content outside the cell; analysis of C2-ceramide as a release-promoting agent

Supernatant samples from 3.2 were analysed by SDS-PAGE. SDS-PAGE was performed using NuPAGE Novex high performance pre-cast gels (4-12% Bis-Tris gradient gel, Invitrogen, Paisley, UK) according to manufacturer's instructions. The samples (20 µl) were mixed with 2.0 µl reducing agent and 6.0 µl sample buffer according to the manufacturer's instructions and subsequently heated for 10 min at 70ºC before loading onto the gel. After electrophoresis, gels were stained using Simply Blue Safe stain (Invitrogen, Paisley, UK) according to manufacturer's instructions.

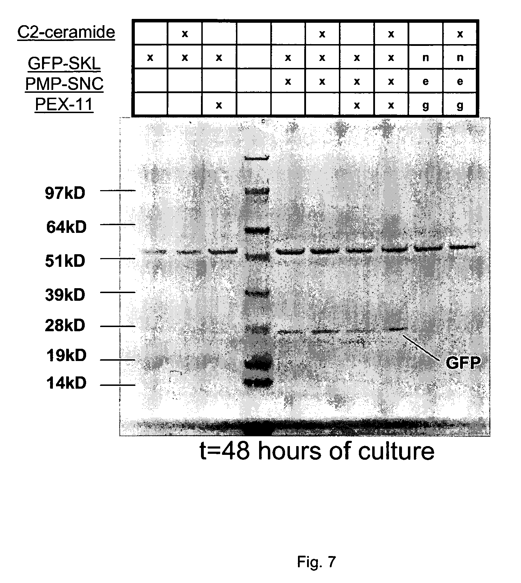

In figure 7, the release of GFP-SKL outside the cell can be clearly observed. All samples contain equal amounts of endogenous protein as observed by the endogenous band at 54 kD. In contrast, lanes, 4, 5, 6 and 7 contain significantly more GFP-SKL as compared to control lanes 1, 2 and 3. Furthermore, it is demonstrated that C2-ceramide further induces the release of peroxisomal content, i.e. GFP-SKL outside the cells; lanes 5 and 7 (MEAM + C2-ceramide) contain more GFP-SKL as compared to lanes 4 and 6, respectively.

### 3.4 Western blot analysis of the release of peroxisomal content outside the cell; analysis of C2-ceramide as a release-promoting agent

Supernatant samples from 3.2 were analysed by Western blot. SDS-PAGE was carried out as in 3.3, Western blot was carried out using the XCell II Blot Module according to manufacturer's instructions (Invitrogen, Paisley, UK). After electrophoresis, the NuPage gel was blotted for 1 hour at 30 volt, on a pre-cut nitrocellulose membrane (Invitrogen). The blot was blocked at room temperature (rT) for 1 hour in Tris Buffered Saline (TBS, 20mM Tris-HCl pH 7.4, 0.9% w/v NaCl; catalogues number: T5912, Sigma) + 2% skim milk. The commercially available anti-GFP antibody (anti-GFP Eurogentec, Belgium catno: MMS-118P) was diluted to 1/10.000 and the blot was incubated for 1 hour with this antibody. Subsequently the blot was washed 3 times for 5 minutes with MilliQ water and TBS + 0.2% skim milk. After washing, the blot was incubated for one hour at room temperature with a 1/5000 dilution of Goat Anti-Rabbit Peroxidase (catalogues number 31460, Pierce Rock Ford Illinois USA). The blot was washed again and immersed with detection fluid (ECL direct nucleic acid labelling and detection system, Amersham biosciences, Buckinghamshire, UK). Finally, the blot was exposed to AGFA Curix Blue HC-S plus film (AGFA Mortsel, Belgium).

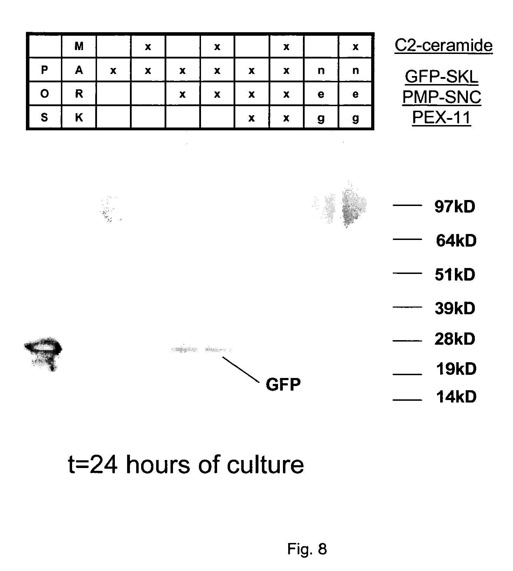

In figure 8, the release of GFP-SKL outside the cell can be clearly observed. In lanes 5, 6, 7 and 8, GFP-SKL is present. In contrast, in lanes 3, 4 and 9, 10, no extracellular GFP-SKL is present.

### 3.5 Analysis of aspecific lysis of A. niger cultures

To demonstrate that the observed release of peroxisomal content as described in 3.1 to 3.4 was not mediated by aspecific lysis, the degree of lysis of the cultures was determined by measurement of the activity of the intra-cellular enzyme acetamidase (amdS) in the culture supernatant. Acetamidase measurement was performed according to Skouloubris et al., Molecular Microbiology (2001) 40 (3), 596-609. Ten µl of supernatant from cultures was added to 100mM acetamide (catalogues number A0500, Sigma Missouri USA) in Phosphate EDTA Buffer (PEB, 100mM Na-Phosphate, pH 7.4, 10mM EDTA). After 90 minutes incubation at 37ºC, 400µl Phenolnitroprusside (catalogus number P6994, Sigma Missouri USA) and 400µl alkaline hypochloride solution (0.2%, catalogue number A1727, Sigma Missouri USA) was added and mixed. The mix was incubated at 55ºC for 6 minutes. Subsequently, the absorption was measured at 635nm in an Ultraspec 2000 UV/VIS Spectrophotometer (Pharmacia Biotech, Sweden) according to the manufacturer's instructions. The amount of amdS in the samples was calculated from a standard curve and designated as relative units/ ml culture supernatant.

The results are depicted in Table 1. It can be clearly observed that after 24 hours of cultivation, no amdS activity is present in the culture supernatants, demonstrating that no lyses has occurred. After 48 hours of cultivation, all samples contain equivalent amounts of amdS activity, demonstrating that the degree of lyses in all cultures is the same. These results clearly show that the observed presence of GFP-SKL in culture supernatants in 3.1 to 3.4 was mediated by active release of peroxisomal content outside the cells and not by a-specific lysis of cells.

**Table 1. Degree of aspecific lysis of cells, depicted as relative amounts of acetamidase (amdS)/ml culture supernatant.**

| ***A. niger* strain** | **24 hours** | **48 hours** |
|---|---|---|
| | **amdS** | **amdS** |
| GFP-SKL | 0.007 | 0.039 |
| GFP-SKL + C2-ceramide | -0.008 | 0.044 |
| GFP-SKL + SNC/PMP | -0.011 | 0.053 |
| GFP-SKL + SNC/PMP + C2-ceramide | -0.011 | 0.056 |
| GFP-SKL + SNC/PMP + PEX-11 | -0.011 | 0.054 |
| GFP-SKL + SNC/PMP + PEX-11 + C2-ceramide | -0.011 | 0.050 |
| Empty control strain | -0.004 | 0.054 |
| Empty control strain + C2-ceramide | 0.001 | 0.052 |

### Example 4. Pmp22 can be utilised to decorate peroxisomes with recombinant (poly)peptides

To demonstrate that Pmp22 can be utilised as a peroxisomal membrane anchor, a chimeric gene was constructed in which Green Fluorescent Protein (GFP) was fused to the N-terminal end of Pmp22 and expressed in *A. niger.* Fluorescence microscopy revealed intense green, spherical shaped, intra-cellular micro bodies.

### 4.1 Cloning and expression of GFP/Pmp22 chimeric construct

GFP, shown as SEQ ID NO: 25 and 26 was fused to peroxisomal membrane protein 22 (Pmp22), shown as SEQ ID NO's: 14, 15 and 16.

GFP DNA (SEQ ID NO: 25) was used as template in a PCR reaction using SEQ ID NO: 27 and SEQ ID NO: 28 to result in SEQ ID NO: 29. In addition, genomic DNA from CBS513.88 was used as template in a PCR reaction using SEQ ID NO: 30 and SEQ ID NO: 31 to result in coding SEQ ID NO: 32. Subsequently, SEQ ID: 29 and SEQ ID: 32 were used as template in a PCR reaction using SEQ ID: 27 and SEQ ID: 31 to result in SEQ ID: 33. The resulting PCR fragment (SEQ ID NO: 33) was digested with restriction enzymes *Pac*l and *Asc*l according to the manufacturers instructions and ligated into a *Pac*I*, Asc*I linearised *A*. *niger* expression vector as depicted in figure 1. This resulted in a construct in which the gene encoding GFP with peroxisomal membrane anchor (GFP/Pmp-22) was placed under control of the glaA promoter. Expression of the gene resulted in GFP with peroxisomal membrane anchor shown as SEQ ID NO: 34. The GFP/Pmp-22 expression vector was used to transform *A. niger.* All PCR reactions, ligations and transformations were performed using classical molecular biology techniques, as described in Molecular Cloning: A Laboratory Manual, Sambrook et al., New York: Cold Spring Harbor Press, 1989.

The resulting *A*. *niger* strains over-expressing GFP/Pmp-22 were cultivated in shake flasks using an orbital shaker at 30ºC, 250 RPM in Minimal Enriched Aspergillus Medium (MEAM, described in patent application WO 98/46772). Control cultures over-expressing GFP with and without a C-terminal linked -SKL (as described in example 2) were cultivated using identical culture conditions. After 18 hours of culture, samples were analysed using a Zeiss fluorescence microscope, using blue light excitation (490nm). Representative samples were used for fluorescence photography using Fujicolor 800ASA colorfilm.

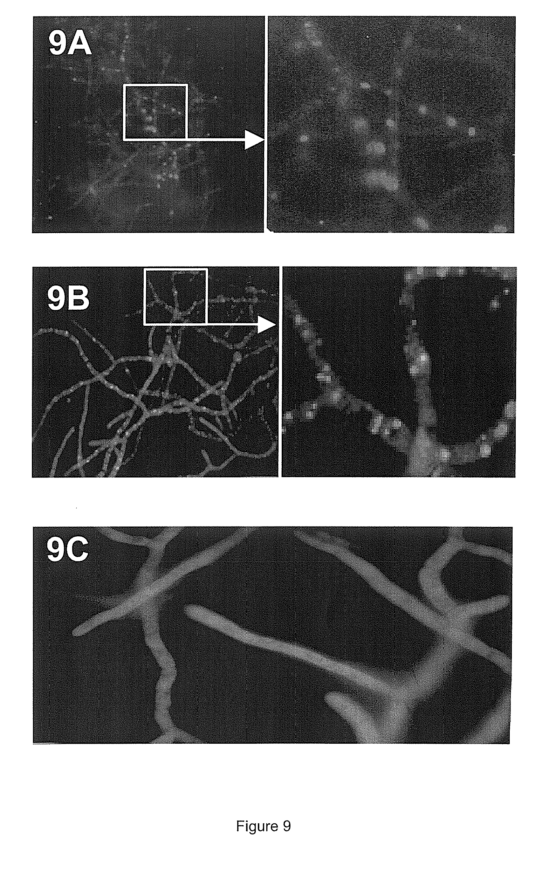

As can be observed in figure 9A, *A*. *niger* over-expressing GFP/Pmp22 chimeric protein shows the same punctuate pattern of intense green, spherical shaped, intra-cellular micro bodies as *A*. *niger* over-expressing GFP-SKL (Figure 9B), where GFP-SKL is targeted to the peroxisome by the C-terminal SKL. In contrast, *A. niger* over-expressing wild-type GFP (i.e. without C-terminal SKL), shows general green fluorescence throughout the whole cytoplasm of the cells (Figure 9C).

The combined results clearly demonstrate that Pmp-22 can be utilised as a peroxisomal membrane anchor to decorate peroxisomes with recombinant (poly)peptides like GFP or the fusing peptides described in this invention.

## Claims

1. A eukaryotic cell containing a peroxisome that is capable to fuse with a membrane-structure of the cell involved in the secretory pathway of the cell.

2. The eukaryotic cell according to claim 1, wherein the membrane-structure of the cell involved in the secretory pathway of the cell is selected from the group consisting of the plasma membrane, the Golgi complex and the Endoplasmic Reticulum.

3. The eukaryotic cell according to claim 1 or 2, wherein a fusing polypeptide or a part thereof is exposed at the surface of the peroxisome.

4. The eukaryotic cell according to claim 3, wherein a part of the fusing polypeptide is exposed that is normally exposed at the surface of a donor membrane-structure.

5. The eukaryotic cell according to claim 3 or 4, wherein the fusing polypeptide or a part thereof is fused to or operatively associated with a peroxisomal membrane polypeptide or a part thereof.

6. The eukaryotic cell according to claim 5, wherein the part of the peroxisomal membrane polypeptide comprises at least one trans-membrane domain.

7. The eukaryotic cell according to any of the claims 3 to 7, wherein the fusing polypeptide is a v-SNARE.

8. The eukaryotic cell according to claim 7, wherein the v-SNARE is Snc1, Snc2 or a homologue thereof, preferably SncA.

9. The eukaryotic cell according to claim 5 or 6, wherein the peroxisomal membrane polypeptide is Pmp22 or a homologue thereof.

10. The eukaryotic cell according to any of the preceding claims, wherein a complementing fusing polypeptide or a part thereof is over-expressed at an acceptor membrane-structure.

11. The eukaryotic cell according to claim 10, wherein the acceptor membrane-structure is the plasma membrane.

12. The eukaryotic cell according to claim 10 or 11, wherein the complementing fusing polypeptide is a t-SNARE.

13. The eukaryotic cell according to claim 3, wherein the cell comprises a nucleic acid construct comprising a nucleic acid sequence encoding a fusing polypeptide or a part thereof operatively associated with a peroxisomal membrane polypeptide or a part thereof

14. The eukaryotic cell according to claim 13, wherein the nucleic acid construct comprises a nucleic acid sequence encoding a chimeric polypeptide with an amino acid sequence according to SEQ ID NO: 24, preferably is a nucleic acid sequence according to SEQ ID NO:23.

15. The eukaryotic cell according to any one of the preceding claims, wherein the cell additionally comprises a nucleic acid construct or an expression vector comprising a DNA sequence encoding a polypeptide of interest operatively associated with a DNA sequence promoting peroxisomal localisation of the polypeptide of interest.

16. The eukaryotic cell according to claim 15, wherein the signal promoting peroxisomal localisation is an amino acid sequence selected from the group consisting of (a) a tripeptide sequence wherein the first amino acid in the N- to C-terminal direction is A, C, H, K, N, P, S or T, the second amino acid in the N- to C-terminal direction is H, K, N, Q, R or S and the third amino acid in the N- to C-terminal direction is A, F, I, L, M or V; and (b) a peptide defined as follows: (R/K) (UV/I/Q) XX (L/V/I/H/Q) (L/S/G/A/K) X(H/Q)(L/A/F), wherein X may be any amino acid.

17. The eukaryotic cell according to any one of preceding claims, wherein the eukaryotic cell is a mammalian, insect, plant, fungus or algal cell.

18. The eukaryotic cell according to claim 16, wherein the fungal cell is a yeast cell, preferably *K. lactis* or *S*. *cerevisiae.*

19. The eukaryotic cell according to claim 16, wherein the fungal cell is a filamentous fungal cell, preferably a cell belonging to a species of an *Aspergillus, Penicillium* or *Trichoderma* genus.

20. The eukaryotic cell according to claim 18, wherein the filamentous fungal cell belongs *Aspergillus niger, Aspergillus oryzae, Trichoderma reesei* or *Penicillium chrysogenum.*

21. A method for production of a polypeptide of interest in the eukaryotic cell according to any one of claims 15 to 20, wherein said polypeptide of interest is present in the peroxisome of the cell, said method comprising culturing the eukaryotic cell in a given fermentation medium, and optionally purifying the compound.

22. The method according to claim 21, wherein the fermentation medium comprises an activator of CAPP, preferably ceramide and/or a substance inducing peroxisome proliferation, preferably oleate.

23. Method according to claim 21 or 22, wherein at least 0.01 g/I of the polypeptide of interest is produced.

24. A polypeptide displaying a v-SNARE function selected from the group consisting of (a) a polypeptide having an amino acid sequence according to SEQ ID NO: 13; (b) a polypeptide having an amino acid sequence that displays a degree of identity of at least 85%, preferably at least 90%, more preferably at least 93%, even more preferably at least 95% even more preferably at least 97%, even more preferably at least 98%, even more preferably at least 99% to the amino acid sequence according to SEQ ID NO: 13; and (c) a functional fragment of the polypeptide defined in (a) or (b)..

25. A peroxisomal membrane polypeptide selected from the group consisting of (a) a polypeptide having an amino acid sequence according to SEQ ID NO: 16; (b) a polypeptide having an amino acid sequence that displays a degree of identity of at least 85%, preferably at least 90%, more preferably at least 93%, even more preferably at least 95% even more preferably at least 97%, even more preferably at least 98%, even more preferably at least 99% to the amino acid sequence according to SEQ ID NO: 16; and (c) a functional fragment of the polypeptide defined in (a) or (b).

26. A chimeric polypeptide suitable to obtain exposure at the surface of a peroxisome of an amino acid sequence corresponding to the amino acid sequence of a fusing polypeptide exposed at the surface of a donor membrane-structure, wherein the chimeric polypetide comprises a fusing polypeptide or part thereof operatively associated with a peroxisomal membrane polypeptide or a part thereof.

27. The chimeric polypeptide according to claim 26 comprising as a fusing polypeptide component an amino acid sequence selected from the group consisting of (a) a sequence corresponding to position 1 to 95 of SEQ ID NO:13 and (b) a homologous sequence displaying a degree of identity of at least 50%, preferably at least 60%, more preferably at least 70%, even more preferably at least 80%, most preferably at least 90%. to the sequence defined in (a).

28. The chimeric polypeptide according to claim 26 comprising as a peroxisomal membrane component an amino acid sequence selected from the group consisting of (a) a sequence corresponding to position 2 to 224 of SEQ ID NO:16, preferably corresponding to a position 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 to position 224 and (b) a homologous sequence displaying a degree of identity of at least 50% preferably at least 60%, more preferably at least 70%, even more preferably at least 80%, most preferably at least 90% to the sequence defined in (a).

29. The chimeric polypeptide according to any one of the claims 26 to 28 having an amino acid sequence according to SEQ ID NO: 24.

30. A polynucleotide comprising a nucleic acid sequence encoding a polypeptide according to any one of the claims 24 to 29.
